# EUROPÄISCHE PATENTSCHRIFT

(11) **EP 3 233 778 B1**
(45) Veröffentlichungstag und Bekanntmachung des Hinweises auf die Patenterteilung: **20.02.2019**
(21) Anmeldenummer: 15817247.8
(22) Anmeldetag: 18.12.2015
(51) Int. Cl.: C07C 45/62, C07C 47/21, C07C 47/02, C07C 29/17, C07C 29/56, C07C 35/12, C07C 35/17

(54) **VERFAHREN ZUR HERSTELLUNG OPTISCH AKTIVER CARBONYLVERBINDUNGEN**
METHOD FOR PRODUCING OPTICALLY ACTIVE CARBONYL COMPOUNDS
PROCÉDÉ DE FABRICATION DE COMPOSÉS CARBONYLE OPTIQUES ACTIFS

(30) Priorität: 19.12.2014 EP 14199410
(43) Veröffentlichungstag der Anmeldung: 25.10.2017
(60) Teilanmeldung: 18206833.8
(73) Patentinhaber: BASF SE, 67056 Ludwigshafen (DE)
(72) Erfinder: SCHELWIES, Mathias, 69115 Heidelberg (DE); PACIELLO, Rocco, 67098 Bad Dürkheim (DE); HEYDRICH, Gunnar, 67117 Limburgerhof (DE); WEGNER, Günter, 67354 Römerberg (DE); TEBBEN, Gerd-Dieter, 68239 Mannheim (DE); HAUBNER, Martin, 69214 Eppelheim (DE); KELLER, Andreas, 67346 Speyer (DE); BEY, Oliver, 67150 Niederkirchen (DE); RENZ, Stephanie, 68723 Schwetzingen (DE); SEEBER, Georg, 67245 Lambsheim (DE)
(86) Internationale Anmeldenummer: PCT/EP2015/080392
(87) Internationale Veröffentlichungsnummer: WO 2016/097242

(56) Entgegenhaltungen:
- WO-A1-2006/040096
- WO-A1-2008/132057
- "Saturated aldehydes selective prodn. - by treating unsaturated aldehyde with hydrogen and carbon monoxide, using catalyst, tertiary phosphine and amine", DERWENT, 26. Dezember 1975 (1975-12-26), XP002364613,

## Beschreibung

Die vorliegende Erfindung betrifft ein Verfahren zur Herstellung einer optisch aktiven Carbonylverbindung durch asymmetrische Hydrierung einer prochiralen α,β-ungesättigter Carbonylverbindung mit Wasserstoff in Gegenwart wenigstens eines im Reaktionsgemisch löslichen, optisch aktiven Übergangsmetall-Katalysators. Speziell betrifft die vorliegende Erfindung ein Verfahren zur Herstellung optisch aktiver Aldehyde oder Ketone, insbesondere Citronellal durch asymmetrische Hydrierung der entsprechenden prochiralen α,β-ungesättigten Aldehyde oder Ketone.

### HINTERGRUND DER ERFINDUNG

Viele optisch aktive Aldehyde und Ketone stellen wertvolle Intermediate in der Synthese höher veredelter chiraler Wert- und Wirkstoffe dar oder sind bereits selber wertvolle Riech- und Aromastoffe.

Die EP-A 0 000 315 betrifft ein Verfahren zur Herstellung von optisch aktivem Citronellal durch Hydrierung von Geranial oder Neral in Gegenwart eines im Reaktionssystem gelösten Katalysatorkomplexes aus Rhodium und einem chiralen Phosphin.

T.-P. Dang et al. beschreiben in J. Mol. Cat., 1982, Band 16, Seiten 51 - 59, ein Verfahren zur homogenkatalytischen Hydrierung α,β-ungesättigter Aldehyde sowie die Anwendung dieses Verfahrens zur Herstellung von optisch aktivem Citronellal. Als Katalysatoren dienten dabei Komplexverbindungen aus einer Rhodiumcarbonyl-Verbindung und einem chiralen Diphosphin.

Auch Chapuis et al. erwähnen in Helv. Chim. Acta, 2001, Band 84, Seiten 230 -242, Fußnote 4, die asymmetrische Hydrierung von Geranial bzw. Neral zu optisch aktivem Citronellal in Gegenwart eines Katalysators aus Rh₄(CO)₁₂ und (R,R)-Chiraphos (2R, 3R)-2,3-bis(diphenylphosphino)butan.

Ein Problem bei der Durchführung mittels löslicher Katalysatoren katalysierter (homogenkatalytischer) Reaktionen besteht in der oft unzureichenden Stabilität der eingesetzten Katalysatorkomplexe bzw. der sich daraus bildenden katalytisch aktiven Metall- bzw. Übergangsmetall-Komplexverbindung. Vor dem Hintergrund des oft hohen Preises derartiger Katalysatoren bzw. Katalysatorvorstufen sind homogenkatalytische Reaktionen mit komplexen Übergangsmetallkatalysatoren nur in speziellen Fällen in wirtschaftlicher Weise im technischen Maßstab anwendbar.

Die JP-A 52078812 beschreibt ein Verfahren zur Hydrierung α,β-ungesättigter Aldehyde wie Crotonaldehyd, Zimtaldehyd oder α-Methylzimtaldehyd an homogenen Rh-Katalysatoren unter Hydroformylierungsbedingungen in Gegenwart eines Triarylphosphins, eines tertiären Amins in stöchiometrischer Menge und Kohlenmonoxid.

Die WO 2006/040096 beschreibt ein Verfahren zur Herstellung optisch aktiver Carbonylverbindungen durch asymmetrische Hydrierung α,β-ungesättigter Carbonylverbindungen mit Wasserstoff in Gegenwart eines im Reaktionsgemisch löslichen, optisch aktiven Übergangsmetall-Katalysators, der wenigstens einen Kohlenmonoxid-Liganden aufweist, das dadurch gekennzeichnet ist, dass man den Katalysator mit einem Kohlenmonoxid und Wasserstoff enthaltenden Gasgemisch vorbehandelt und/oder die asymmetrische Hydrierung in Gegenwart von dem Reaktionsgemisch zusätzlich zugeführtem Kohlenmonoxid durchführt.

Die WO 2008/132057 beschreibt ebenfalls ein Verfahren zur Herstellung optisch aktiver Carbonylverbindungen durch asymmetrische Hydrierung α,β-ungesättigter Carbonylverbindungen, welches auf dem in der WO 2006/040096 offenbarten Verfahren beruht. Zur besseren Kontrolle der Kohlenmonoxidkonzentration im Reaktionsgemisch während der Hydrierung beinhaltet dieses Verfahren zusätzlich die Maßgaben, dass die Vorbehandlung der Katalysator-Vorstufe mit einem Gasgemisch umfassend 20 bis 90 Vol.-% Kohlenmonoxid, 10 bis 80 Vol-% Wasserstoff und 0 bis 5 Vol.-% weiterer Gase, wobei sich die genannten Volumenanteile zu 100 Vol.-% ergänzen, bei einem Druck von 5 bis 100 bar durchgeführt, von dem so erhaltenen Katalysator vor Einsatz in der asymmetrischen Hydrierung überschüssiges Kohlenmonoxid abtrennt und die asymmetrische Hydrierung in Gegenwart von Wasserstoff mit einem Kohlenmonoxidgehalt von 100 bis 1200 ppm durchführt wird.

Insbesondere bei den letztgenannten Verfahren zur asymmetrischen Hydrierung α,β-ungesättigter Carbonylverbindungen konnte gegenüber herkömmlichen Verfahren die Stabilität bzw. die Lebensdauer der eingesetzten Katalysatorkomplexe bzw. der sich daraus bildenden katalytisch aktiven Metall- bzw. Übergangsmetall-Komplexverbindung wesentlich verbessert werden. Aufgrund moderater und schwankender Katalysator-Aktivitäten sind diese Verfahren hinsichtlich ihrer Umsatzraten verbesserungswürdig, insbesondere wenn diese im technischen Maßstab durchgeführt werden.

### ZUSAMMENFASSUNG DER ERFINDUNG

Aufgabe der vorliegenden Erfindung war es, ein verbessertes Verfahren zur homogenkatalytischen asymmetrischen Hydrierung α,β-ungesättigter Aldehyde oder Ketone bereitzustellen, das sich durch eine verbesserte Katalysatoraktivität bei zugleich hoher Enantiomerenselektivität auszeichnet, um hohe Umsatzraten bei hoher Produktselektivität zu erreichen. Weiterhin soll sich das katalytische System durch eine hohe Stabilität und damit durch eine erhöhte Lebensdauer der katalytisch aktiven, in optisch aktiver Form einzusetzenden Übergangsmetall-Komplexverbindung auszeichnen. Das Verfahren soll sich dadurch für Anwendungen im technischen Maßstab in besonderem Maße eignet.

Es wurde überaschenderweise gefunden, dass die katalytische Aktivität der zur homogenkatalytischen asymmetrischen Hydrierung prochiraler α,β-ungesättigter Carbonylverbindungen eingesetzten optisch aktiver Übergangsmetall-Katalysatoren, die Rhodium als katalytisch aktives Übergangsmetall enthalten, durch die Zugabe einer Phosphinverbindung der allgemeinen Formel (I), wie im folgenden definiert, deutlich gesteigert werden kann, ohne deren Stabilität und Selektivität in signifikanter Weise nachteilig zu beeinflussen.

Die vorliegende Erfindung betrifft somit ein Verfahren zur Herstellung einer optisch aktiven Carbonylverbindung durch asymmetrische Hydrierung einer prochiralen α,β-ungesättigter Carbonylverbindung mit Wasserstoff in Gegenwart wenigstens eines im Reaktionsgemisch löslichen, optisch aktiven Übergangsmetall-Katalysators, der Rhodium als katalytisch aktives Übergangsmetall und einen chiralen, zweizähnigen Bisphosphin-Liganden aufweist, wobei das Reaktionsgemisch während der Hydrierung der prochiralen α,β-ungesättigter Carbonylverbindung zusätzlich wenigstens eine Verbindung der allgemeinen Formel (I) enthält: 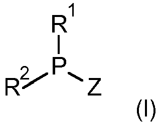 worin
- R¹, R²:: gleich oder verschieden sind und für C₆- bis C₁₀-Aryl stehen, das unsubstituiert ist oder einen oder mehrere, z.B. 1, 2, 3, 4 oder 5, Substituenten trägt, die ausgewählt sind unter C₁- bis C₆-Alkyl, C₃- bis C₆-Cycloalkyl, C₆- bis C₁₀-Aryl, C₁- bis C₆-Alkoxy und Amino;
- Z: für eine Gruppe CHR³R⁴ oder Aryl steht, das unsubstituiert ist oder einen oder mehrere, z.B. 1, 2, 3, 4 oder 5, Substituenten trägt, die ausgewählt sind unter C₁- bis C₆-Alkyl, C₃- bis C₆-Cycloalkyl, C₆- bis C₁₀-Aryl, C₁- bis C₆-Alkoxy und Amino, worin
- R³: für C₁- bis C₄-Alkyl, C₁- bis C₄-Alkoxy-C₁- bis C₄-alkyl, C₃- bis C₆-Cycloalkyl oder C₆- bis C₁₀-Aryl steht, wobei eine oder zwei nicht benachbarte CH₂-Gruppen in C₃- bis C₆-Cycloalkyl auch durch ein bzw. zwei Sauerstoffatome ersetzt sein können;
- R⁴: für C₁- bis C₄-Alkyl, das unsubstituiert ist oder eine Gruppe P(=O)R^{4a}R^{4b} trägt, C₁- bis C₄-Alkoxy, C₁- bis C₄-Alkoxy-C₁- bis C₄-alkyl, C₃- bis C₆-Cycloalkyl oder C₆- bis C₁₀-Aryl steht, wobei eine oder zwei nicht benachbarte CH₂-Gruppen in C₃- bis C₆-Cycloalkyl auch durch ein Sauerstoffatom ersetzt sein können und wobei C₃-bis C₆-Cycloalkyl und C₆- bis C₁₀-Aryl unsubstituiert sind oder einen oder mehrere, z.B. 1, 2, 3, 4 oder 5, Substituenten tragen, die ausgewählt sind unter C₁- bis C₄-Alkyl, C₁- bis C₄-Alkoxy und Amino;
oder
- R³, R⁴:: zusammen mit dem C-Atom, an das sie gebunden sind, für C₄- bis C₈-Cycloalkyl steht, worin eine oder zwei nicht benachbarte CH₂-Gruppen in C₃- bis C₆-Cycloalkyl auch durch ein bzw. zwei Sauerstoffatome ersetzt sein können und wobei C₃- bis C₆-Cycloalkyl unsubstituiert ist oder einen oder mehrere, z.B. 1, 2, 3, 4 oder 5, Subsitutenten trägt, die ausgewählt sind unter C₁- bis C₄-Alkyl, C₁- bis C₄-Alkoxy und A-P(=O)R^{4a}R^{4b},
wobei A für eine chemische Bindung oder für C₁- bis C₄-Alkylen steht; und
R^{4a}, R^{4b} gleich oder verschieden sind und für Phenyl stehen, das unsubstituiert ist oder einen oder mehrere Substituenten trägt, die ausgewählt sind unter C₁- bis C₆-Alkyl, C₃- bis C₆-Cycloalkyl, C₆- bis C₁₀-Aryl, C₁- bis C₆-Alkoxy und Amino.

Speziell betrifft die vorliegende Erfindung ein Verfahren zur Herstellung optisch aktiver Aldehyde oder Ketone, insbesondere Citronellal durch asymmetrische Hydrierung der entsprechenden prochiralen α,β-ungesättigten Aldehyde oder Ketone.

### AUSFÜHRLICHE BESCHREIBUNG DER ERFINDUNG

Das erfindungsgemäße Verfahren eignet sich zur Herstellung optisch aktiver Carbonylverbindungen wie Aldehyden, Ketonen, Estern, Lactonen oder Lactamen durch asymmetrische, d.h. enantioselektive Hydrierung der entsprechenden Carbonylverbindungen, die in α,β-Position zur Carbonylgruppe eine ethylenische Doppelbindung aufweisen. Erfindungsgemäß hydriert man die zur Carbonylgruppe α,β-ständige ethylenische Doppelbindung in Gegenwart eines im Reaktionsgemisch löslichen, optisch aktiven Übergangsmetallkatalysators, der Rhodium als katalytisch aktives Übergangsmetall enthält, sowie einer Phosphinverbindung der allgemeinen Formel (I) zu einer Kohlenstoff-Kohlenstoff-Einfachbindung, wobei das dadurch in β-Position neu geschaffene tetraedrische Kohlenstoffatom vier verschiedene Substituenten trägt und in nichtracemischer Form erhalten wird. Unter dem Begriff asymmetrische Hydrierung ist demgemäß im Rahmen der vorliegenden Erfindung eine Hydrierung zu verstehen, bei der die beiden enantiomeren Formen des Hydrierproduktes nicht zu gleichen Teilen erhalten werden.

Bei den in den vorstehenden und nachfolgenden Formeln angegebenen Definitionen der Variablen werden Sammelbegriffe verwendet, die allgemein repräsentativ für die jeweiligen Substituenten stehen. Die Bedeutung Cₙ- bis Cₘ- gibt die jeweils mögliche Anzahl von Kohlenstoffatomen in dem jeweiligen Substituenten oder Substituententeil an.

Im Rahmen der vorliegenden Erfindung umfasst der Ausdruck "Alkyl" unverzweigte oder verzweigte Alkylgruppen mit 1 bis 4, 6, 12 oder 25 C-Atomen. Dazu zählen beispielsweise C₁- bis C₆-Alkyl, wie Methyl, Ethyl, Propyl, Isopropyl, n-Butyl, Isobutyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl und dergleichen. Bevorzugt handelt es sich bei "Alkyl" um unverzweigte oder verzweigte C₁- bis C₆-Alkylgruppen.

Im Rahmen der vorliegenden Erfindung umfasst der Ausdruck "Cycloalkyl" cyclische, gesättigte Kohlenwasserstoffgruppen mit 3 bis 6, 12 oder 25 Kohlenstoffringgliedern, z.B. C₃-C₈-Cycloalkyl wie Cyclopropyl, Cyclobutyl, Cyclopentyl, Cyclohexyl, Cycloheptyl und Cyclooctyl, oder C₇-C₁₂-Bicycloalkyl.

Im Rahmen der vorliegenden Erfindung steht der Ausdruck "Alkoxy" für eine über ein Sauerstoff gebundene Alkylgruppe mit 1 bis 6 C-Atomen, z.B. C₁- bis C₆-Alkoxy, wie Methoxy, Ethoxy, n-Propoxy, 1-Methylethoxy, Butoxy, 1-Methylpropoxy, 2-Methylpropoxy, 1,1-Dimethylethoxy, Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy oder 1-Ethyl-2-methylpropoxy. Bevorzugt handelt es sich bei "Alkoxy" um C₁- bis C₄-Alkoxy.

Im Rahmen der vorliegenden Erfindung umfasst der Ausdruck "Alkenyl" unverzweigte oder verzweigte Kohlenwasserstoffreste mit 2 bis 4, 6, 12 oder 25 C-Atomen, die wenigstens eine Doppelbindung, beispielsweise 1, 2, 3, oder 4 Doppelbindungen enthalten. Dazu zählen beispielsweise C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl. Bevorzugt handelt es sich bei "Alkenyl" um unverzweigte C₂- bis C₁₂-Alkenylgruppen oder verzweigte C₃- bis C₁₂-Alkenylgruppen mit jeweils 1 bis 3 Doppelbindungen, besonders bevorzugt um unverzweigte C₂- bis C₆-Alkenyylgruppen oder verzweigte C₃- bis C₆-Alkenylgruppen mit jeweils einer Doppelbindung.

Im Rahmen der vorliegenden Erfindung bezieht sich der Ausdruck "Alkylen" auf zweiwertige Kohlenwasserstoffreste mit 2 bis 25 Kohlenstoffatomen. Die zweiwertigen Kohlenwasserstoffreste können unverzweigt oder verzweigt sein. Dazu zählen beispielsweise C₂-C₁₆-Alkylengruppen, wie 1,4-Butylen, 1,5-Pentylen, 2-Methyl-1,4-butylen, 1,6-Hexylen, 2-Methyl-1,5-pentylen, 3-Methyl-1,5-pentylen, 1,7-Heptylen, 2-Methyl-1,6-hexylen, 3-Methyl-1,6-hexylene, 2-Ethyl-1,5-pentylen, 3-Ethyl-1,5-pentylen, 2,3-Dimethyl-1,5-pentylen, 2,4-Dimethyl-1,5-pentylen, 1,8-Octylen, 2-Methyl-1,7-heptylen, 3-Methyl-1,7-heptylen, 4-Methyl-1,7-heptylen, 2-Ethyl-1,6-hexylen, 3-Ethyl-1,6-hexylen, 2,3-Dimethyl-1,6-hexylen, 2,4-Dimethyl-1,6-hexylen, 1,9-Nonylen, 2-Methyl-1,8-Octylen, 3-Methyl-1,8-Octylen, 4-Methyl-1,8-Octylen, 2-Ethyl-1,7-heptylen, 3-Ethyl-1,7-heptylen, 1,10-Decylen, 2-Methyl-1,9-nonylen, 3-Methyl-1,9-nonylen, 4-Methyl-1,9-nonylen, 5-Methyl-1,9-nonylen, 1,11-Undecylen, 2-Methyl-1,10-decylen, 3-Methyl-1,10-decylen, 5-Methyl-1,10-decylen, 1,12-Dodecylen, 1,13-Tridecylen, 1,14-Tetradecylen, 1,15-Pentadecylen, 1,16-Hexadecylen und dergleichen. Bevorzugt handelt es sich bei "Alkylen" um unverzweigte C₂- bis C₁₂-Alkylengruppen oder verzweigte C₃- bis C₁₂-Alkylengruppen, insbesondere um unverzweigte C₂- bis C₆-Alkylengruppen oder verzweigte C₃- bis C₆-Alkylengruppen.

Bei den einfach oder mehrfach verzweigten oder substituierten Alkylengruppen kann das Kohlenstoffatom am Verzweigungspunkt oder die Kohlenstoffatome an den jeweiligen Verzweigungspunkten bzw. die einen Substituenten tragenden Kohlenstoffatome, unabhängig voneinander eine R-, oder eine S-Konfiguration oder beide Konfigurationen zu gleichen oder unterschiedlichen Anteilen aufweisen.

Im Rahmen der vorliegenden Erfindung bezieht sich der Ausdruck "Alkenylen" auf zweiwertige Kohlenwasserstoffreste mit 2 bis 25 Kohlenstoffatomen, welche unverzweigt oder verzweigt sein können, wobei die Hauptkette eine oder mehrere Doppelbindungen, beispielsweise 1, 2, oder 3 Doppelbindungen aufweist. Dazu zählen beispielsweise C₂- bis C₁₈-Alkenylengruppen, wie Ethylen, Propylen, 1-, 2-Butylen, 1-, 2-Pentylen, 1-, 2-, 3-Hexylen, 1,3-Hexadienylen, 1,4-Hexadienylen, 1-, 2-, 3-Heptylen, 1,3-Heptadienylen, 1,4-Heptydienylen, 2,4-Heptadienylen, 1-, 2-, 3-Octenylen, 1,3-Octadienylen, 1,4-Octadienylen, 2,4-Octadienylen, 1-, 2-, 3-Nonenylen, 1-, 2-, 3-, 4-, 5-Decenylen, 1-, 2-, 3-, 4-, 5-Undecenylen, 2-, 3-, 4-, 5-, 6-Dodecenylen, 2,4-Dodecadienylen, 2,5-Dodecadienylen, 2,6-Dodecadienylen, 3-, 4-, 5-, 6-Tridecenylen, 2,5-Tridecadienylen, 4,7-Tridecadienylen, 5,8-Tridecadienylen, 4-, 5-, 6-, 7-Tetradecenylen, 2,5-Tetradecadienylen, 4,7-Tetradecadienylen, 5,8-Tetradecadienylen, 4-, 5-, 6-, 7-Pentadecenylen, 2,5-Pentadecadienylen, 4,7-Pentadecadienylen, 5,8-Pentadecadienylen, 1,4,7-Pentadecatrienylen, 4,7,11-Pentadecatrienylen, 4,6,8-Pentadecatrienylen, 4-, 5-, 6-, 7-, 8-Hexadecenylen, 2,5-Hexadecadienylen, 4,7-Hexadecadienylen, 5,8-Hexadecadienylen, 2,5,8-Hexadecatrienylen, 4,8,11-Hexadecatrienylen, 5,7,9-Hexadecatrienylen, 5-, 6-, 7-, 8-Heptadecenylen, 2,5-Heptadecadienylen, 4,7-Heptadecadienylen, 5,8-Heptadecadienylen, 5-, 6-, 7-, 8-, 9-Octadecenylen, 2,5-Octadecadienylen, 4,7-Octadecadienylen, 5,8-Octadecadienylen und dergleichen. Bevorzugt handelt es sich bei "Alkenylen" um unverzweigte C₃- bis C₁₂-Alkenylengruppen oder verzweigte C₄- bis C₁₂-Alkenylengruppen mit jeweils einer oder zwei Doppelbindungen, insbesondere um unverzweigte C₃- bis C₈-Alkenylengruppen mit einer Doppelbindung.

Die Doppelbindungen in den Alkenylengruppen können unabhängig voneinander in der E- als auch in Z-Konfiguration oder als Mischung beider Konfigurationen vorliegen.

Im Rahmen der vorliegenden Erfindung umfasst der Ausdruck " Halogen" Fluor, Chlor, Brom und lod, bevorzugt Fluor, Chlor oder Brom.

Im Rahmen der vorliegenden Erfindung umfasst der Ausdruck "Aryl" ein bis dreikernige aromatisches Ringsystem enthaltend 6 bis 14 Kohlenstoffringglieder. Dazu zählen beispielsweise C₆- bis C₁₀-Aryl, wie Phenyl oder Naphthyl.

Im Rahmen der vorliegenden Erfindung umfasst der Ausdruck "Hetaryl" ein bis dreikernige aromatisches Ringsystem enthaltend 6 bis 14 Kohlenstoffringglieder, wobei ein oder mehrere, beispielsweise 1, 2, 3, 4, 5 oder 6, Kohlenstoffatome durch ein Stickstoff-, Sauerstoff- und/oder Schwefelatom substituiert sind. Dazu zählen beispielsweise C₃- bis C₉-Hetarylgruppen, wie 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl, 1,3,4-Triazol-2-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl, 2-Indolyl, 3-Indolyl, 4-Indolyl, 5-Indolyl, 6-Indolyl, 7-Indolyl und dergleichen. Bevorzugt handelt es sich bei "Hetaryl" um C₅- bis C₆-Hetaryl.

Im Rahmen der vorliegenden Erfindung umfasst der Ausdruck "Aralkyl" ein über eine unverzweigte oder verzweigte C₁- bis C₆-Alkylgruppe gebundenes, ein bis zweikerniges aromatisches Ringsystem, enthaltend 6 bis 10 Kohlenstoffringglieder. Dazu zählen beispielsweise C₇- bis C₁₂-Aralkyl, wie Phenylmethyl, 1-Phenylethyl, 2-Phenylethyl, 1-Phenylpropyl, 2-Phenylpropyl, 3-Phenylpropyl und dergleichen.

Im Rahmen der vorliegenden Erfindung umfasst der Ausdruck "Aralkyl" ein bis zweikernige aromatisches Ringsysteme, enthaltend 6 bis 10 Kohlenstoffringglieder, welche mit einem oder mehreren, beispielsweise 1, 2 oder 3, unverzweigten oder verzweigten C₁- bis C₆-Alkylresten substituiert ist. Dazu zählen z.B. C₇- bis C₁₂-Alkylaryl, wie 1-Methylphenyl, 2-Methylphenyl, 3-Methylphenyl, 1-Ethylphenyl, 2-Ethylphenyl, 3-Ethylphenyl, 1-Propylphenyl, 2-Propylphenyl, 3-Propylphenyl, 1-iso-Propylphenyl, 2-iso-Propylphenyl, 3-iso-Propylphenyl, 1-Butylphenyl, 2-Butylphenyl, 3-Butylphenyl, 1-iso-Butylphenyl, 2-iso-Butylphenyl, 3-iso-Butylphenyl, 1-sec-Butylphenyl, 2-sec-Butylphenyl, 3-sec-Butylphenyl, 1-tert-Butylphenyl, 2-tert-Butylphenyl, 3-tert-Butylphenyl, 1-(1-pentenyl)phenyl, 2-(1-pentenyl)phenyl, 3-(1-pentenyl)phenyl, 1-(2-pentenyl)phenyl, 2-(2-pentenyl)phenyl, 3-(2-pentenyl)phenyl, 1-(3-pentenyl)phenyl, 2-(3-pentenyl)phenyl, 3-(3-pentenyl)phenyl, 1-(1-(2-methylbutyl))phenyl, 2-(1-(2-methylbutyl))phenyl, 3-(1-(2-methylbutyl))phenyl, 1-(2-(2-methylbutyl))phenyl, 2-(2-(2-methylbutyl))phenyl, 3-(2-(2-methylbutyl))phenyl, 1-(3-(2-methylbutyl))phenyl, 2-(3-(2-methylbutyl))phenyl, 3-(3-(2-methylbutyl))phenyl, 1-(4-(2-methylbutyl))phenyl, 2-(4-(2-methylbutyl))phenyl, 3-(4-(2-methylbutyl))phenyl, 1-(1-(2,2-Dimethylpropyl))phenyl, 2-(1-(2,2-Dimethylpropyl))phenyl, 3-(1-(2,2-Dimethylpropyl))phenyl, 1-(1-hexenyl)phenyl, 2-(1-hexenyl)phenyl, 3-(1-hexenyl)phenyl, 1-(2-hexenyl)phenyl, 2-(2-hexenyl)phenyl, 3-(2-hexenyl)phenyl, 1-(3-hexenyl)phenyl, 2-(3-hexenyl)phenyl, 3-(3-hexenyl)phenyl, 1-(1-(2-Methylpentenyl))phenyl, 2-(1-(2-Methylpentenyl))phenyl, 3-(1-(2-Methylpentenyl))phenyl, 1-(2-(2-Methylpentenyl))phenyl, 2-(2-(2-Methylpentenyl))phenyl, 3-(2-(2-Methylpentenyl))phenyl, 1-(3-(2-Methylpentenyl))phenyl, 2-(3-(2-Methylpentenyl))phenyl, 3-(3-(2-Methylpentenyl))phenyl, 1-(4-(2-Methylpentenyl))phenyl, 2-(4-(2-Methylpentenyl))phenyl, 3-(4-(2-Methylpentenyl))phenyl, 1-(5-(2-Methylpentenyl))phenyl, 2-(5-(2-Methylpentenyl))phenyl, 3-(5-(2-Methylpentenyl))phenyl, 1-(1-(2,2-Dimethylbutenyl))phenyl, 2-(1-(2,2-Dimethylbutenyl))phenyl, 3-(1-(2,2-Dimethylbutenyl))phenyl, 1-(3-(2,2-Dimethylbutenyl))phenyl, 2-(3-(2,2-Dimethylbutenyl))phenyl, 3-(3-(2,2-Dimethylbutenyl))phenyl, 1-(4-(2,2-Dimethylbutenyl))phenyl, 2-(4-(2,2-Dimethylbutenyl))phenyl, 3-(4-(2,2-Dimethylbutenyl))phenyl und dergleichen.

In einer bevorzugten Ausführungsform des erfindungsgemäßen Verfahrens werden Verbindungen der Formel (I) eingesetzt, worin Z in Formel (I) für eine Gruppe CHR³R⁴ steht. Hierunter sind solche Verbindungen der allgemeinen Formel (I) bevorzugt, worin die Variablen R¹, R², R³, R⁴ unabhängig voneinander und insbesondere gemeinsam die folgenden Bedeutungen aufweisen:
- R¹, R²:: sind gleich oder verschieden und stehen für Phenyl, das unsubstituiert ist oder 1, 2 oder 3 Substituenten trägt, die ausgewählt sind unter Methyl und Methoxy, wobei R¹ und R² jeweils insbesondere für unsubstituiertes Phenyl stehen;
- R³: steht für C₁- bis C₄-Alkyl, insbesondere für Methyl;
- R⁴: steht für C₁- bis C₄-Alkyl, das unsubstituiert ist oder eine Gruppe P(=O)R^{4a}R^{4b} trägt, wobei R⁴ insbesondere für Methyl oder Ethyl steht oder eine Gruppe CH₂-P(=O)R^{4a}R^{4b} oder CH(CH₃)-P(=O)R^{4a}R^{4b} bedeutet;
oder
- R³, R⁴:: zusammen mit dem C-Atom, an das sie gebunden sind, für C₃- bis C₈-Cycloalkyl steht, worin eine oder zwei CH₂-Gruppen durch ein bzw. 2 nicht benachbarte Sauerstoffatome ersetzt sein können und wobei C₃- bis C₆-Cycloalkyl unsubstituiert ist oder eine Gruppe A-P(=O)R^{4a}R^{4b} trägt und/oder 1, 2, 3 oder 4 Methylgruppen als Substituenten aufweist, wobei R³ und R⁴ zusammen mit dem Kohlenstoffatom, an das sie gebunden sind, insbesondere für Cyclopentyl, Oxolan-3-yl, 2,2-Dimethyl-1,3-dioxolan-4-yl oder Cyclohexyl stehen, oder R³ und R⁴, zusammen mit dem Kohlenstoffatom, an das sie gebunden sind, für Bicyclo[2.2.1]heptan-1-yl stehen können, dass in der 2-Postion eine Gruppe P(=O)R^{4a}R^{4b} aufweist;
wobei
- A: für eine chemische Bindung, CH₂ oder CH(CH₃) steht; und
- R^{4a}, R^{4b}:: gleich oder verschieden sind und für C₆- bis C₁₀-Aryl und insbesondere Phenyl stehen, das unsubstituiert ist oder 1, 2 oder 3 Substituenten trägt, die ausgewählt sind unter Methyl und Methoxy, wobei R^{4a} und R^{4b} insbesondere für Phenyl stehen, das unsubstituiert ist oder 1, 2 oder 3 Substituenten trägt, die ausgewählt sind unter Methyl und Methoxy, wobei besonders bevorzugt R^{4a} und R^{4b} jeweils für unsubstituiertes Phenyl stehen.

In einer besonders bevorzugten Ausführungsform des erfindungsgemäßen Verfahrens werden Verbindungen der Formel (I) eingesetzt, worin Z in Formel (I) für eine Gruppe CHR³R⁴ steht und worin die Variablen R¹, R², R³, R⁴ unabhängig voneinander und insbesondere gemeinsam die folgenden Bedeutungen aufweisen:
- R¹, R²:: sind gleich oder verschieden und stehen für Phenyl, das unsubstituiert ist oder 1, 2 oder 3 Substituenten trägt, die ausgewählt sind unter Methyl und Methoxy, wobei R¹ und R² jeweils insbesondere für unsubstituiertes Phenyl stehen;
- R³: steht für C₁- bis C₄-Alkyl, insbesondere für Methyl;
- R⁴: steht für C₁- bis C₄-Alkyl, das eine Gruppe P(=O)R^{4a}R^{4b} trägt, wobei und insbesondere eine Gruppe CH₂-P(=O)R^{4a}R^{4b} oder CH(CH₃)-P(=O)R^{4a}R^{4b} bedeutet.
wobei
- R^{4a}, R^{4b}:: gleich oder verschieden sind und für Phenyl stehen, das unsubstituiert ist oder 1, 2 oder 3 Substituenten trägt, die ausgewählt sind unter Methyl und Methoxy, wobei besonders bevorzugt R^{4a} und R^{4b} jeweils für unsubstituiertes Phenyl stehen.

In dieser besonders bevorzugten Ausführungsform des erfindungsgemäßen Verfahrens wird insbesondere eine Verbindung der Formel (I) eingesetzt, worin
- R¹, R²:: für unsubstituiertes Phenyl stehen;
- R³: für Methyl steht;
- R⁴: eine Gruppe CH(CH₃)-P(=O)R^{4a}R^{4b} bedeutet, worin R^{4a} und R^{4b} jeweils für unsubstituiertes Phenyl stehen.

Hierbei handelt es sich um die Verbindung (2-(Diphenylphosphoryl)-1-methylpropyl))-diphenylphosphan, einschließlich dessen (R,R)-Enantiomeren (= (*R,R*)-Chiraphosmonooxid) und dessen (S,S)-Enantiomeren (= (*S,S*)-Chiraphosmonooxid) sowie dessen Racemat (Verbindungen (I-1)).

Falls die Reste R³ und R⁴ in der allgemeinen Formel (I) unterschiedliche Bedeutung haben oder gemeinsam mit dem Kohlenstoffatom, an das sie gebunden sind, für einen bicyclischen und/oder substituierten Cycloalkylrest stehen, kann das Kohlenstoffatom, welches die Reste R³ und R⁴ trägt, eine (R)- oder (S)-Konfiguration aufweisen. Diese Verbindungen der allgemeinen Formel (I) können als reine (R)- oder reine (S)-Stereoisomere oder als Mischungen davon vorliegen. In der Regel wird man in diesen Fällen die reinen (R)- und (S)-Stereoisomere einsetzen, wobei auch etwaige Stereoisomerengemische für den Einsatz in dem vorliegenden Verfahren geeignet sind.

Unter einem reinen Stereoisomeren versteht man hier und im Folgenden chirale Substanzen, die bezüglich des gewünschten Stereoisomeren einen Enantiomerenüberschuss (ee) von wenigstens 80 %ee, insbesondere wenigstens 90 %ee und speziell wenigstens 95 %ee aufweisen.

In einer anderen Ausführungsform des erfindungsgemäßen Verfahrens werden Verbindungen der Formel (I) eingesetzt, worin Z in Formel (I) für Aryl steht, das unsubstituiert ist oder einen oder mehrere, z.B. 1, 2, 3, 4 oder 5 Substituenten trägt, die ausgewählt sind unter C₁- bis C₆-Alkyl, C₃- bis C₆-Cycloalkyl, C₆- bis C₁₀-Aryl, C₁- bis C₆-Alkoxy und Amino,. Hierunter sind solche Verbindungen der allgemeinen Formel (I) bevorzugt, worin die Variablen R¹, R², Z unabhängig voneinander und insbesondere gemeinsam die folgenden Bedeutungen aufweisen:
- Z: steht für Phenyl, das unsubstituiert ist oder 1, 2 oder 3 Substituenten trägt, die ausgewählt sind unter Methyl und Methoxy;
- R¹, R²:: sind gleich oder verschieden und stehen für Phenyl, das unsubstituiert ist oder 1, 2 oder 3 Substituenten trägt, die ausgewählt sind unter Methyl und Methoxy, wobei R¹ und R² jeweils insbesondere für unsubstituiertes Phenyl stehen.

Besonders bevorzugte Verbindungen der allgemeinen Formel (I) sind beispielsweise:
- (2-(Diphenylphosphoryl)-1-methylpropyl))-diphenylphosphan, einschließlich dessen (R,R)-Enantiomeren (= (*R*,*R*)-Chiraphosmonooxid) und dessen (S,S)-Enantiomeren (= (*S*,*S*)-Chiraphosmonooxid) sowie dessen Racemats (Verbindungen (I-1)),
- Cyclopentyldiphenylphosphin (Verbindung (I-2),
- 2-Butyldiphenylphosphin (Verbindung (I-3),
- Cyclohexyldiphenylphosphin (Verbindung (I-4),
- Isopropyldiphenylphosphin (Verbindung (I-5),
- [5-(Diphenylphosphanylmethyl)-2,2-dimethyl-1,3-dioxolan-4-yl]methyl-diphenyl-phosphanmonoxid, einschließlich dessen *(4S,5S)*- und *(4R,5R)*-Enantiomeren und dessen Racemats (Verbindungen (I-6),
- [5-(1-Diphenylphosphanylethyl)-2,2-dimethyl-1,3-dioxolan-4-yl]ethyl-diphenyl-phosphanmonoxid, einschließlich dessen *(4S,5S)-* und *(4R,5R)*-Enantiomeren und dessen Racemats (Verbindungen (I-7),
- [2-Diphenylphosphanylcyclohexyl]-diphenyl-phosphanmonoxid, einschließlich dessen *(1S,2S)-* und *(1R,2R)*-Enantiomeren und dessen Racemats (Verbindungen (I-8),
- [4-Diphenylphosphanyltetrahydrofuran-3-yl]-diphenylphosphanmonoxid, einschließlich dessen *(3S,4S)-* und *(3R,4R)*-Enantiomeren und dessen Racemats (Verbindungen (I-9),
- [2-Diphenylphosphanyl-3-bicyclo[2.2.1]hept-5-enyl]-diphenyl-phosphanmonoxid, einschließlich deren *(1S,2R,3R,4R)-, (1R,2S,3R,4R)-, (1S,2R,3S,4S)-, (1R,2S,3S,4S)*-Isomeren, und deren Enantiomeren- und Diastereomerengemischen (Verbindungen (I-10),
- [1-Benzyl-4-diphenylphosphanyl-pyrrolidin-3-yl]-diphenyl-phosphanmonoxid, einschließlich dessen *(3S,4S)-* und *(3R,4R)*-Enantiomeren und dessen Racemats (Verbindungen (I-11),
- [3-Diphenylphosphanyl-1-methyl-butyl]-diphenyl-phosphanmonoxid, einschließlich dessen *(1S,3S)-* und *(1R,3R)*-Enantiomeren und dessen Racemats (Verbindungen (I-12),
- und deren Mischungen.

Ebenfalls geeignet ist Triphenylphosphin (Verbindung (1-13)) und dessen Gemische mit einer oder mehrerer der vorgenannten Verbindungen der Formel (I), z.B. mit einer der Verbindungen (I-1) bis (I-12).

Die Verbindung der Formel (I) wird in dem erfindungsgemäßen Verfahren in der Regel in einer Menge von 0,01 bis 1 Mol, bevorzugt 0,02 bis 0,8 Mol, besonders bevorzugt 0,03 bis 0,7 Mol und insbesondere in einer Menge von 0,05 bis 0,6 Mol pro Mol Rhodium einsetzt.

Als Einsatzstoffe in dem erfindungsgemäßen Verfahren eignen sich in der Regel alle Arten von prochiralen Carbonylverbindungen, die an der 2-Position eine Doppelbindung aufweisen. Bevorzugt handelt es sich bei der prochiralen α,β-ungesättigten Carbonylverbindung um prochirale, α,β-ungesättigte Keton oder insbesondere um prochirale, α,β-ungesättigte Aldehyde.

Dementsprechend eignet sich das erfindungsgemäße Verfahren bevorzugt zur Herstellung optisch aktiver Aldehyde oder Ketone durch asymmetrische Hydrierung prochiraler α,β-ungesättigter Aldehyde oder Ketone. Das erfindungsgemäße Verfahren eignet sich besonders bevorzugt zur Herstellung optisch aktiver Aldehyde durch asymmetrische Hydrierung prochiraler α,β-ungesättigter Aldehyde.

In einer bevorzugten Ausführungsform des erfindungsgemäßen Verfahrens ist die prochirale α,β-ungesättigte Carbonylverbindung ausgewählt unter Verbindungen der allgemeinen Formel (II) 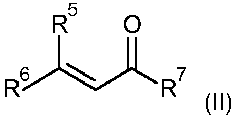 worin
- R⁵, R⁶: voneinander verschieden sind und jeweils für einen unverzweigten, verzweigten oder cyclischen Kohlenwassestoffrest mit 1 bis 25 Kohlenstoffatomen steht, der gesättigt ist oder eine oder mehrere, z.B. 1, 2, 3, 4 oder 5, vorzugsweise nicht konjugierte ethylenische Doppelbindungen aufweist, und der unsubstituiert ist oder einen oder mehrere, z.B. 1, 2, 3 oder 4, gleiche oder verschiedene Substituenten trägt, die ausgewählt sind unter OR⁸, NR^{9a}R^{9b}, Halogen, C₆- bis C₁₀-Aryl und C₃- bis C₉-Hetaryl;
- R⁷: für Wasserstoff oder für einen unverzweigten, verzweigten oder cyclischen Kohlenwassestoffrest mit 1 bis 25 Kohlenstoffatomen steht, der gesättigt ist oder eine oder mehrere, z.B. 1, 2, 3, 4 oder 5, vorzugsweise nicht konjugierte ethylenische Doppelbindungen aufweist, und der unsubstituiert ist oder einen oder mehrere, z.B. 1, 2, 3 oder 4, gleiche oder verschiedene Substituenten trägt, die ausgewählt sind unter OR⁸, NR^{9a}R^{9b}, Halogen, C₆- bis C₁₀-Aryl und C₃- bis C₉-Hetaryl;
oder
- R⁷: gemeinsam mit einem der Reste R⁵ oder R⁶ auch eine 3 bis 25-gliedrige Alkylengruppe bedeuten kann, worin 1, 2, 3 oder 4 nicht benachbarte CH₂-Gruppen durch O oder N-R^{9c} ersetzt sein können, wobei die Alkylengruppe gesättigt ist oder eine oder mehrere, z.B. 1, 2, 3, 4 oder 5, vorzugsweise nicht konjugierte ethylenische Doppelbindungen aufweist, und wobei die Alkylengruppe unsubstituiert ist oder einen oder mehrere, z.B. 1, 2, 3 oder 4, gleiche oder verschiedene Substituenten trägt, die ausgewählt sind unter OR⁸, NR^{9a}R^{9b}, Halogen, C₁- bis C₄-Alkyl, C₆- bis C₁₀-Aryl und C₃- bis C₉-Hetaryl, wobei zwei Substituenten auch gemeinsam für eine 2 bis 10-gliedrige Alkylengruppe stehen können, wobei die 2- bis 10-gliedrige Alkylengruppe gesättigt ist oder eine oder mehrere, z.B. 1, 2, 3 oder 4, nicht konjugierte ethylenische Doppelbindungen aufweist, und wobei die 2- bis 10-gliedrige Alkylengruppe unsubstituiert ist oder einen oder mehrere, z.B. 1, 2, 3 oder 4, gleiche oder verschiedene Substituenten trägt, die ausgewählt sind unter OR⁸, NR^{9a}R^{9b}, Halogen, C₆- bis C₁₀-Aryl und C₃- bis C₉-Hetaryl;
wobei
- R⁸: für Wasserstoff, C₁- bis C₆-Alkyl, C₆- bis C₁₀-Aryl, C₇- bis C₁₂-Aralkyl oder C₇- bis C₁₂-Alkylaryl steht;
- R^{9a}, R^{9b}: jeweils unabhängig voneinander Wasserstoff, C₁- bis C₆-Alkyl, C₆- bis C₁₀-Aryl, C₇- bis C₁₂-Aralkyl oder C₇- bis C₁₂-Alkylaryl bedeuten oder
- R^{9a} und R^{9b}: gemeinsam auch eine Alkylenkette mit 2 bis 5 Kohlenstoffatomen, die durch N oder O unterbrochen sein kann, bedeuten können; und
- R^{9c}: für Wasserstoff, C₁- bis C₆-Alkyl, C₆- bis C₁₀-Aryl, C₇- bis C₁₂-Aralkyl oder C₇- bis C₁₂-Alkylaryl steht.

Bei den in den Definitionen der Reste R⁵, R⁶ und R⁷ genannten unverzweigten, verzweigten oder cyclischen Kohlenwassestoffresten mit 1 bis 25 Kohlenstoffatomen, handelt es sich in der Regel um unverzweigte C₁- bis C₂₅-Alkylgruppen, unverzweigte C₂- bis C₂₅-Alkenylgruppen, unverzweigte C₄- bis C₂₅-Alkadienylgruppen, verzweigte C₃- bis C₂₅-Alkylgruppen, verzweigte C₃- bis C₂₅-Alkenylgruppen, verzweigte C₅- bis C₂₅-Alkadienylgruppen sowie um C₃- bis C₂₅-Cycloalkylgruppen oder um C₃- bis C₂₄-Cycloalkylgruppen, die durch ein oder mehrere, z.B. durch 1, 2, 3 oder 4 C₁- bis C₄-Alkylgruppen, wie zuvor definiert, substituiert sind. Zu den cyclischen Kohlenwasserstoffreste zählen auch solche cyclischen Kohlenwasserstoffreste, die einen Phenylring aufweisen, der gegebenenfalls einen oder mehrere, z.B. 1, 2, 3, 4, 5 oder 6 C₁-C₄-Alkylgruppen trägt, wobei der Phenylring unmittelbar an die ethylenisch ungesättigte Doppelbindung oder die Carbonylgruppe in Formel (II) gebunden ist oder über eine C₁-C₆-Alkylengruppe gebunden ist.

Unter einer Alkenylgruppe versteht man einen linearen oder verzweigten aliphatischen Kohlenwasserstoffrest, der einfach ungesättigt ist. Unter einer Alkdienylgruppe versteht man einen linearen oder verzweigten aliphatischen Kohlenwasserstoffrest, der zweifach ungesättigt ist. Bei den in der Definition des Restes R⁷ genannten 3- bis 25-gliedrigen Alkylengruppen, welche gesättigt sind, handelt es sich in der Regel um unverzweigte oder verzweigte C₃- bis C₂₅-Alkylengruppen, wie zuvor definiert.

Bei den in der Definition des Restes R⁷ genannten 3- bis 25-gliedrigen Alkylengruppen, welche eine oder mehrere, z.B. 1, 2, 3 oder 4, nicht konjugierte ethylenische Doppelbindungen aufweisen, handelt es sich in der Regel um unverzweigte oder verzweigte C₃- bis C₂₅-Alkenylengruppen, wie zuvor definiert.

Vorzugsweise steht einer der Reste R⁵, R⁶ für Methyl oder Ethyl, insbesondere für Methyl und der andere Rest für einen unverzweigten, verzweigten oder cyclischen Kohlenwassestoffrest mit 3 bis 25 Kohlenstoffatomen, der gesättigt ist oder eine oder mehrere, z.B. 1, 2, 3, 4 oder 5, vorzugsweise nicht konjugierte ethylenische Doppelbindungen aufweist, und der unsubstituiert ist oder einen oder mehrere, z.B. 1, 2, 3 oder 4, gleiche oder verschiedene Substituenten trägt, die ausgewählt sind unter OR⁸, NR^{9a}R^{9b}, Halogen, C₆- bis C₁₀-Aryl und C₃- bis C₉-Hetaryl.

Insbesodere steht einer der Reste R⁵, R⁶ für Methyl oder Ethyl, insbesondere für Methyl und der andere Rest für einen unverzweigten, verzweigten oder cyclischen Kohlenwassestoffrest mit 3 bis 25 Kohlenstoffatomen, der gesättigt ist oder eine oder mehrere, z.B. 1, 2 oder 3, vorzugsweise nicht konjugierte ethylenische Doppelbindungen aufweist.

R⁷ steht insbesondere für Wasserstoff.

In einer ganz bevorzugten Ausführungsform des erfindungsgemäßen Verfahrens ist die die prochirale α,β-ungesättigte Carbonylverbindung ausgewählt unter Verbindungen der allgemeinen Formel (IIa) und (IIb) 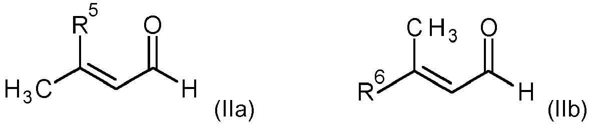 worin
- R⁵, R⁶: jeweils für einen unverzweigten oder verzweigten Kohlenwassestoffrest mit 2 bis 25, insbesondere mit 3 bis 20 Kohlenstoffatomen steht, der gesättigt ist oder 1, 2, 3, 4 oder 5 nicht konjugierte ethylenische Doppelbindungen aufweist

Entsprechend lassen sich mit dem erfindungsgemäßen Verfahren durch asymmetrische Hydrierung prochiraler α,β-ungesättigter Aldehyde oder Ketone der allgemeinen Formeln (II), (IIa) und (IIb) die entsprechenden α,β-gesättigten Aldehyde oder Ketone der Formel (III) in optisch aktiver Form herstellen, wobei das Kohlenstoffatom, welches die Reste R⁵ und R⁶ trägt, das durch die Hydrierung erzeugte Asymmetriezentrum darstellt.

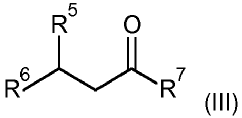

In Formel (III) weisen R⁵, R⁶ und R⁷ die für Formel (II), insbesondere die für die Formeln (IIa) und (IIb) genannten Bedeutungen auf.

Durch die erfindungsgemäße asymmetrische, d.h. enantioselektive Hydrierung der α,β-ungesättigten Aldehyde der Formeln (IIa) oder (IIb) sind die entsprechenden α,β-gesättigten Aldehyde zugänglich. Die Verbindungen der Formeln (IIa) und (IIb) stellen E/Z-Doppelbindungsisomere zueinander dar. Prinzipiell sind die entsprechend optisch aktiven Aldehyde ausgehend von beiden Doppelbindungsisomeren der Formeln (IIa) und (IIb) zugänglich. Je nach Wahl der enantiomeren Form des Katalysators, d.h. je nach Wahl des (+)- bzw. (-)-Enantiomeren des Katalysators bzw. des (+)- bzw. (-)-Enantiomeren des eingesetzten chiralen Liganden, erhält man in erfindungsgemäßer Weise aus dem eingesetzten E- oder Z-Doppelbindungsisomeren bevorzugt eines der Enantiomere des optisch aktiven Aldehyds. Gleiches gilt für die vorstehend genannten Substrat bzw. Produktklassen. Prinzipiell lassen sich auch Gemische der beiden Doppelbindungsisomere in erfindungsgemäßer Weise umsetzen. Man erhält auf diese Weise Gemische der beiden Enantiomere der gewünschten Zielverbindung.

Als Beispiele optisch aktiver Aldehyde oder Ketone, die sich mit dem erfindungsgemäßen Verfahren herstellen lassen, seien die folgenden Verbindungen genannt:

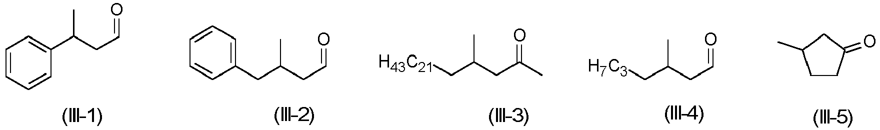

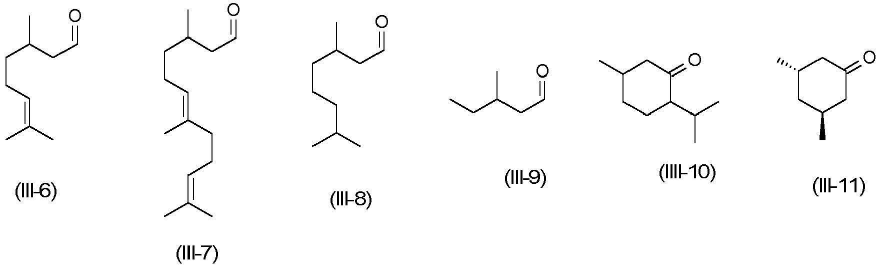

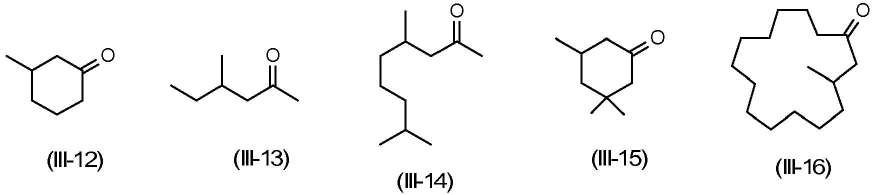

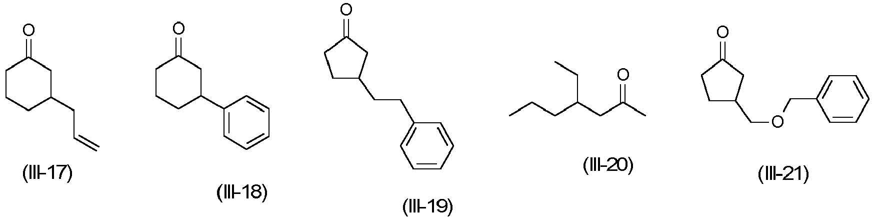

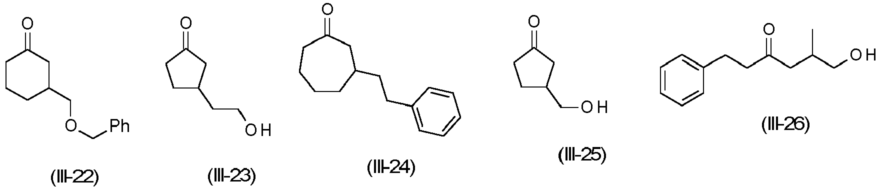

Besonders bevorzugt eignet sich das erfindungsgemäße Verfahren zur Herstellung von optisch aktivem Citronellal der Formel (IV) 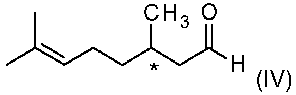 worin * das Asymmetriezentrum bezeichnet; durch asymmetrische Hydrierung von Geranial der Formel (IIa-1) oder von Neral der Formel (IIb-1)

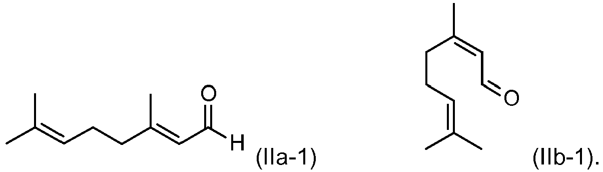

Auch Gemische von Geranial und Neral lassen sich in erfindungsgemäßer Weise umsetzen, wobei man, wie vorstehend beschrieben Gemische von D- bzw. L-Citronellal erhält, die optisch aktiv sind, falls die beiden Enantiomere nicht zu gleichen Teilen darin vorliegen.

Im Rahmen des erfindungsgemäßen Verfahrens insbesondere bevorzugt ist die erfindungsgemäße Herstellung von D-Citronellal durch asymmetrische Hydrierung von Neral bzw. Geranial.

Das erfindungsgemäße Herstellverfahren wird in Gegenwart eines im Reaktionsgemisch löslichen, optisch aktiven Übergangsmetall-Katalysators, der Rhodium als katalytisch aktives Übergangsmetall enthält, durchgeführt.

Derartige Katalysatoren sind beispielsweise erhältlich durch Reaktion mindestens einer geeigneten, im Reaktionsgemisch löslichen Rhodiumverbindung mit einem optisch aktiven Liganden, der mindestens ein Phosphor- und/oder Arsenatom aufweist. Geeignete Rhodiumverbindungen sind insbesondere solche, die im gewählten Reaktionsmedium löslich sind, wie beispielsweise Rhodium (0), Rhodium(I)-, Rhodium(II)- und Rhodium(III)-Salze wie z.B. Rhodium(III)-chlorid, Rhodium(III)-bromid, Rhodium(III)-nitrat, Rhodium(III)-sulfat, Rhodium(II)- oder Rhodium(III)-oxid, Rhodium(II)- oder Rhodium(III)-acetat, Rhodium(II)- oder Rhodium(III)-carboxylat, Rh(acac)₃, [Rh(cod)Cl]₂, [Rh(cod)₂]BF₄, Rh₂(OAc)₄, Bis(ethylen)rhodium(I)acac, Rh(CO)₂acac, [Rh(cod)OH]₂, [Rh(cod)OMe]₂, Rh₄(CO)₁₂ oder Rh₆(CO)₁₆, wobei "acac" für einen Acetylacetonat-Liganden, "cod" für einen Cyclooctadien-Liganden und "OAc" für einen Acetat-Liganden steht.

Vorzugsweise wird als Rhodiumverbindung Rh(OAc)₃, [Rh(cod)Cl]₂, Rh(CO)₂ acetylacetonat, [Rh(cod)OH]₂, [Rh(cod)OMe]₂, Rh₄(CO)₁₂ oder Rh₆(CO)₁₆, Rh₂(OAc)₄ und Bis(ethylen)rhodium(I) acetylacetonat eingesetzt. Insbesondere wird als Rhodiumverbindung Rh(CO)₂acac, Rh₂(OAc)₄ und Bis(ethylen)rhodium(I) acetylacetonat eingesetzt.

Die genannten und weitere geeignete Rhodium-verbindungen bzw. Rhodiumkomplexe sind bekannt und in der Literatur hinreichend beschrieben oder können vom Fachmann analog zu den bereits bekannten Verbindungen hergestellt werden.

Die genannten Rhodiumverbindungen bzw. Rhodiumkomplexe setzt man erfindungsgemäß üblicherweise in einer Menge von etwa 0,001 bis etwa 1 mol-%, bevorzugt von etwa 0,002 bis etwa 0,5 mol-%, insbesondere von etwa 0,005 bis etwa 0,2 mol-% (bezogen auf die enthaltenen Übergangsmetallatome) im Verhältnis zur Menge an zu hydrierendem Substrat ein.

Bei unter kontinuierlichen Bedingungen durchgeführten Umsetzungen wählt man das Verhältnis von eingesetzter Menge an Übergangsmetallverbindung als Vorläufer des erfindungsgemäßen homogenen Katalysators zur Menge an zu hydrierendem Substrat vorteilhaft so, dass eine Katalysatorkonzentration im Bereich von etwa 100 ppm bis 10000 ppm, insbesondere im Bereich von etwa 200 ppm bis 5000 ppm eingehalten wird.

Die genannten Rhodiumverbindungen bzw. Rhodiumkomplexe werden erfindungsgemäß mit einer weiteren Verbindung in Kontakt gebracht, die optisch aktiv, bevorzugt im wesentlichen enantiomerenrein ist (d.h. einen Enantiomerenüberschuss von mindestens 90 %ee, insbesondere mindestens 95 %ee oder mindestens 98 %ee oder mindestens 99 %ee aufweist) und zwei Phosphoratome aufweist. Diese als chiraler Ligand zu bezeichnende Verbindung bildet im Reaktionsgemisch oder in einer der Hydrierung vorgelagerten Präformierungsstufe mit der eingesetzten Übergangsmetall-Verbindung den erfindungsgemäß einzusetzenden Übergangsmetall-Katalysator.

Erfindungsgemäß werden solche chiralen Liganden eingesetzt, die zwei Phosphoratome aufweisen und mit dem eingesetzten Übergangsmetall Chelatkomplexe bilden und die im Folgenden als zweizähnige Bisphosphin-Liganden bezeichnet werden.

Als chirale zweizähnige Bisphosphin-Liganden eignen sich im Rahmen der vorliegenden Erfindung solche Verbindungen, wie sie beispielsweise in: I. Ojima (Hrsg.), Catalytic Asymmetric Synthesis, Wiley-VCh, 2. Auflage, 2000 oder in E. N. Jacobsen, A. Pfaltz, H. Yamamoto (Hrsg.), Comprehensive Asymmetric Catalysis, 2000, Springer oder in W. Tang, X. Zhang, Chem. Rev. 2003, 103, 3029-3069 beschrieben sind.

Beispielhaft seien die folgenden Verbindungen als erfindungsgemäß bevorzugt einsetzbare chirale Liganden (1) bis (90) sowie deren Enantiomere angeführt:

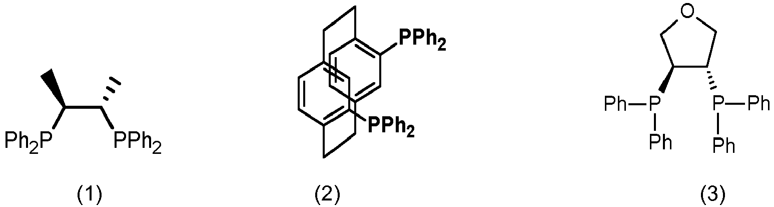

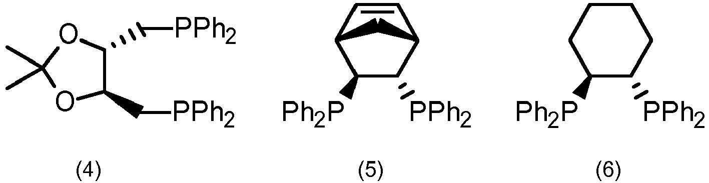

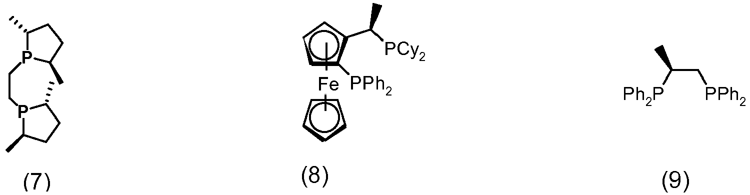

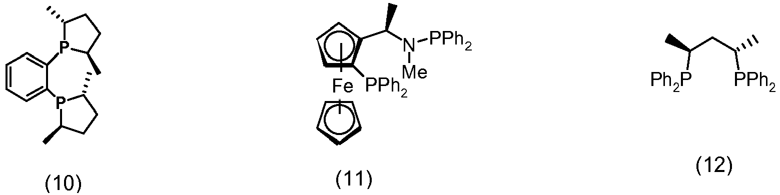

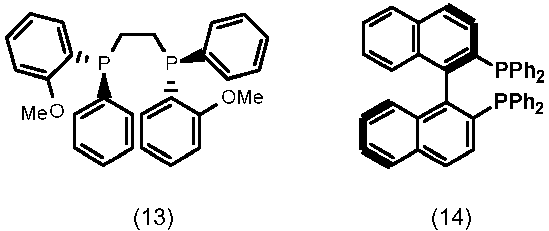

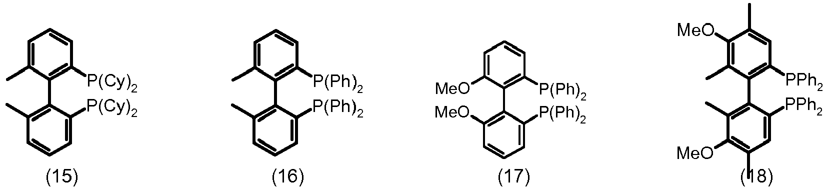

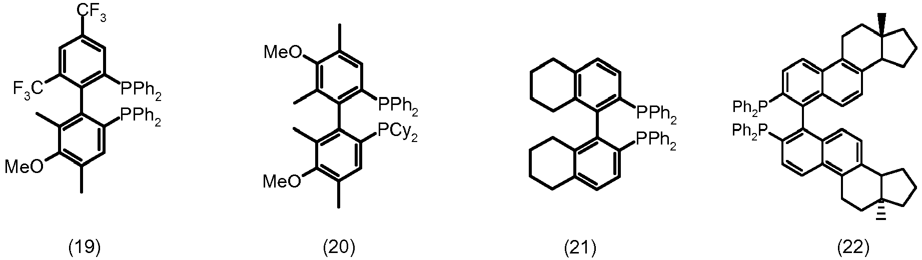

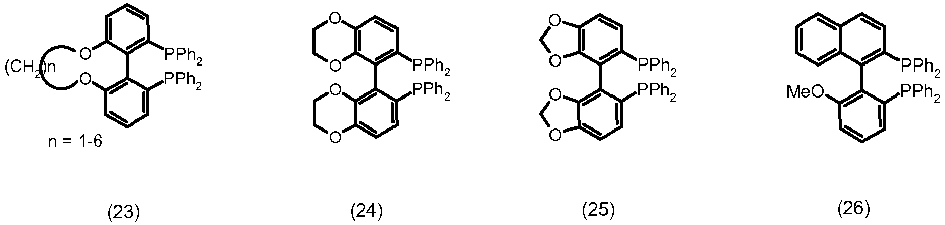

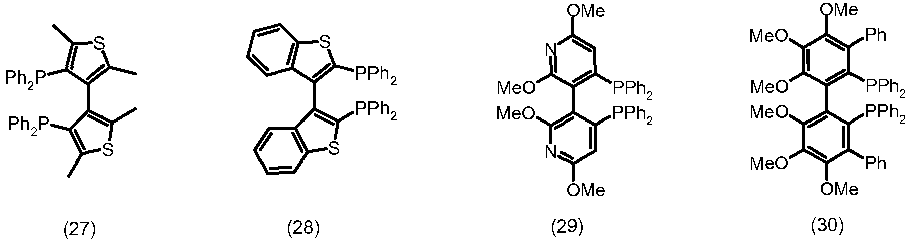

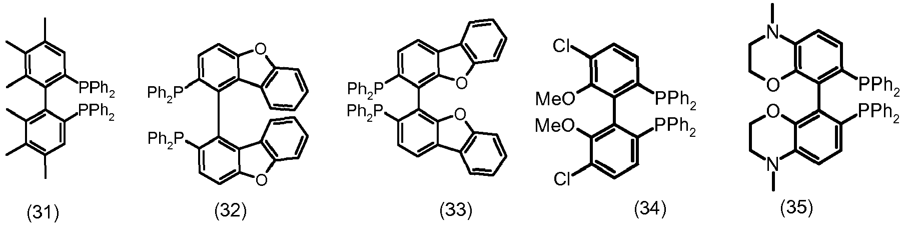

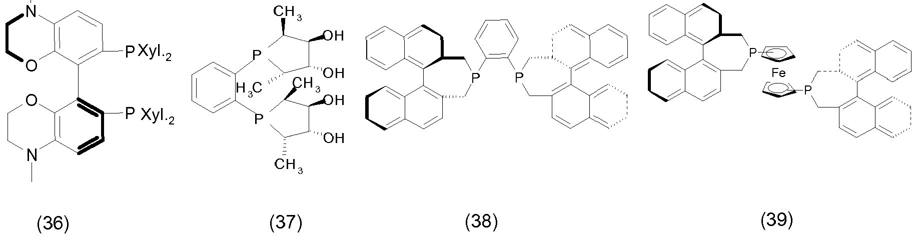

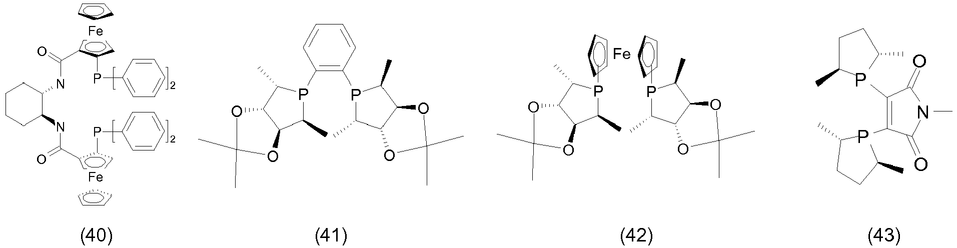

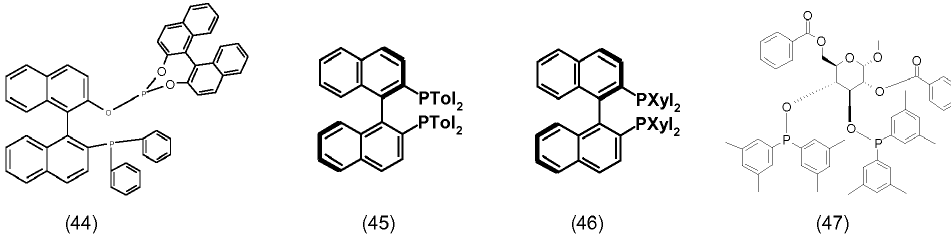

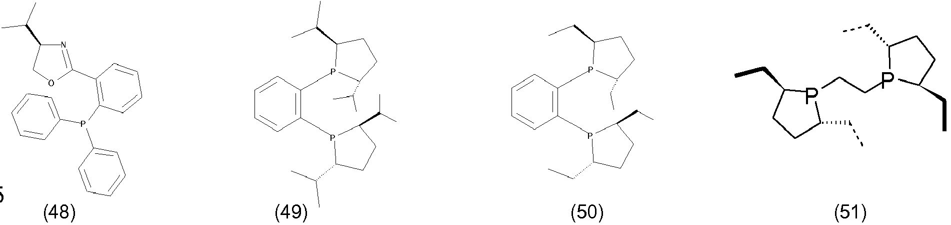

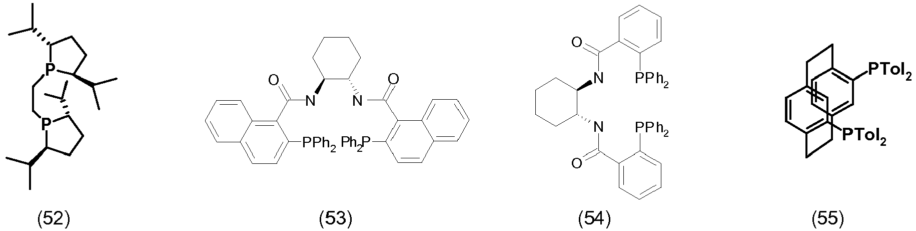

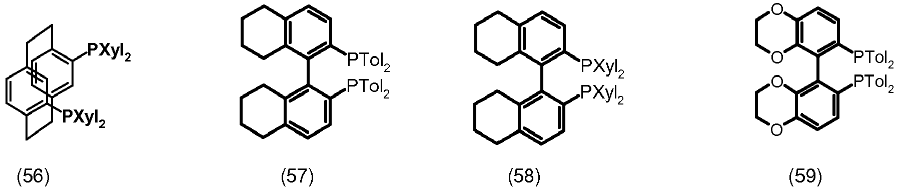

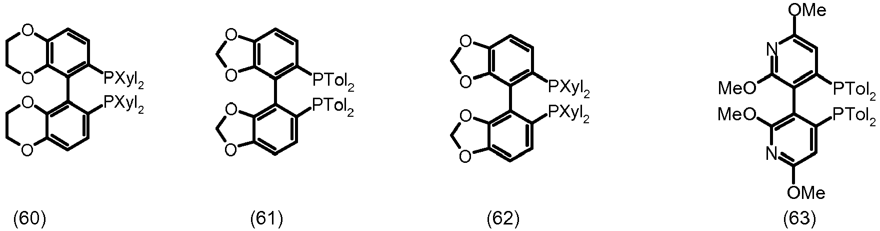

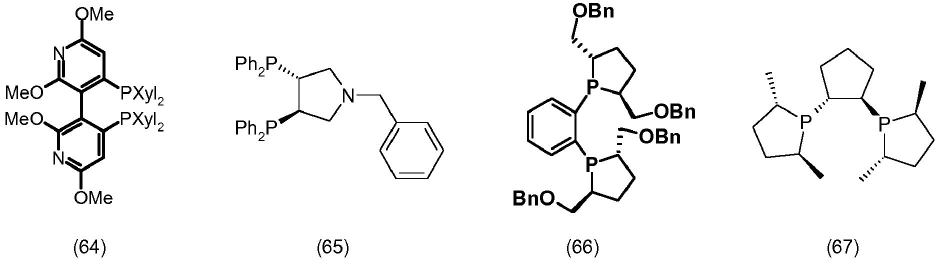

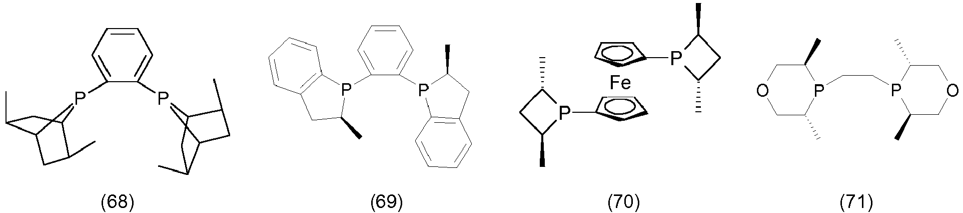

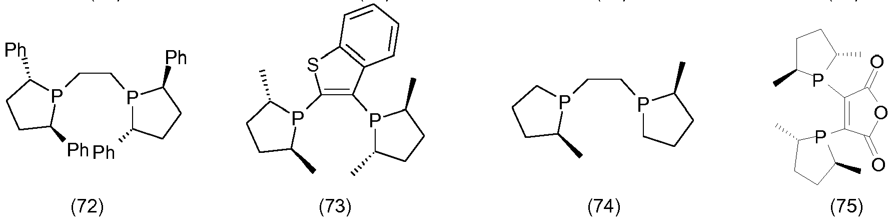

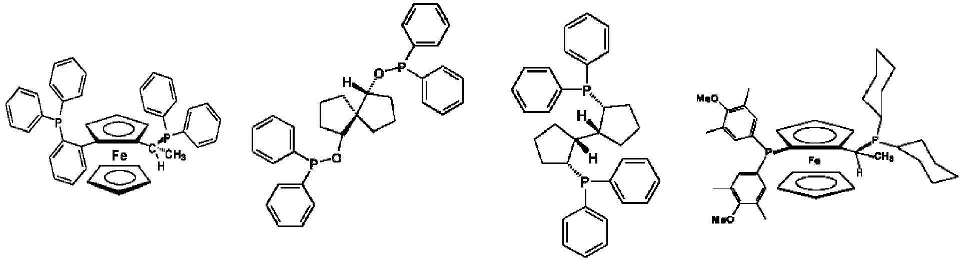

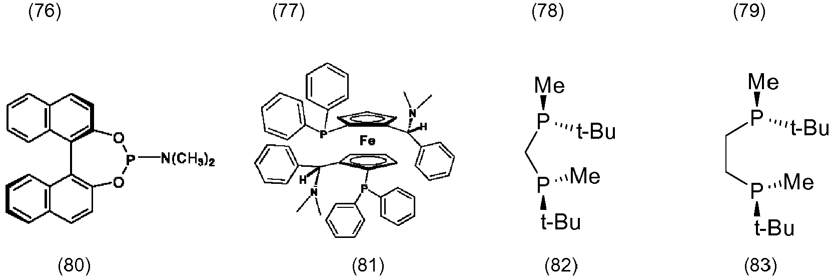

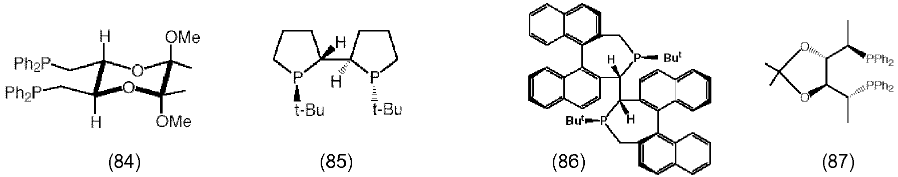

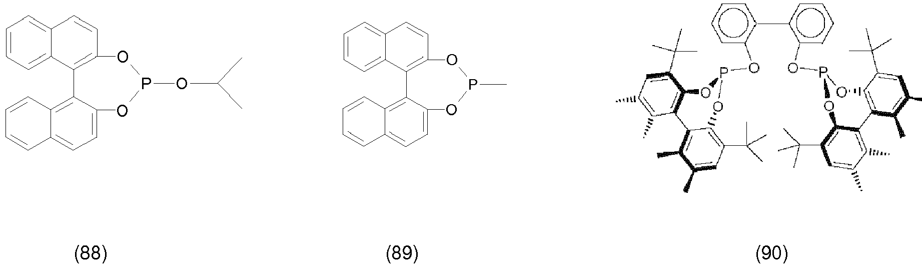

In den Formeln (1) bis (90) ist unter "Ph" Phenyl, "Cy" Cyclohexyl, "Xyl" Xylyl, "Tol" p-Tolyl und "Bn" Benzyl zu verstehen.

Erfindungsgemäß besonders bevorzugte Liganden sind die der Strukturformeln (1) bis (14) sowie (37), (38), (41), (43), (49), (50), (51), (52), (65), (66), (67), (68), (69), (71), (72), (73), (74), (75), (83), (84), (85), (86), (87).

Insbesondere bevorzugte chirale, zweizähnige Bisphosphin-Liganden sind solche der allgemeinen Formeln (IX), (X) oder (XI) 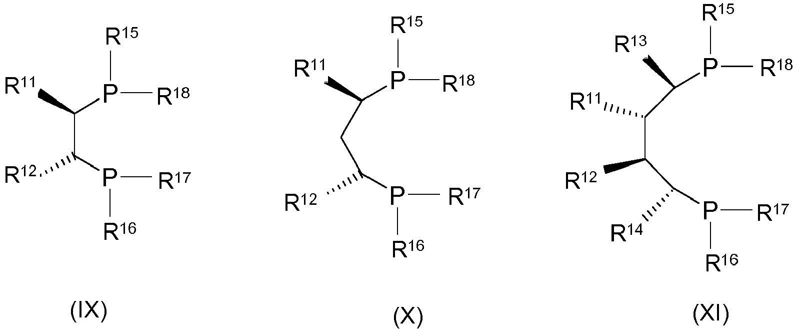 worin
- R¹¹, R¹²:: jeweils unabhängig voneinander für einen unverzweigten, verzweigten oder cyclischen Kohlenwasserstoffrest mit 1 bis 20 Kohlenstoffatomen stehen, der gesättigt ist oder eine oder mehrere, in der Regel 1 bis etwa 4, nicht konjugierte, ethylenische Doppelbindungen aufweisen kann und der unsubstituiert ist oder einen oder mehrere, in der Regel 1 bis 4, gleiche oder verschiedene Substituenten trägt, die ausgewählt sind unter OR¹⁹, NR²⁰R²¹, Halogen, C₆-C₁₀-Aryl und C₃-C₉-Hetaryl, oder
- R¹¹ und R¹²: gemeinsam auch eine 2 bis 10-gliedrige Alkylengruppe oder eine 3 bis 10-gliedrige Cycloalkylengruppe bedeuten kann worin 1, 2, 3 oder 4 nicht benachbarte CH₂-Gruppen durch O oder N-R^{9c} ersetzt sein können, wobei die Alkylengruppe und die Cycloalkylengruppe gesättigt sind oder eine oder zwei, nicht konjugierte ethylenische Doppelbindungen aufweisen, und wobei die Alkylengruppe und die Cycloalkylengruppe unsubstituiert sind oder einen oder mehrere gleiche oder verschiedene Substituenten tragen, die ausgewählt sind unter C₁- bis C₄-Alkyl;
- R¹³, R¹⁴:: jeweils unabhängig voneinander Wasserstoff oder geradkettiges oder verzweigtes C₁- bis C₄-Alkyl bedeuten und
- R¹⁵, R¹⁶, R¹⁷, R¹⁸:: gleich oder verschieden sind und für C₆- bis C₁₀-Aryl stehen, das unsubstituiert ist oder einen oder mehrere Substituenten trägt, die ausgewählt sind unter C₁- bis C₆-Alkyl, C₃- bis C₆-Cycloalkyl, C₆- bis C₁₀-Aryl, C₁- bis C₆-Alkoxy und Amino;
- R¹⁹, R²⁰, R²¹:: jeweils unabhängig voneinander Wasserstoff, C₁-C₄-Alkyl, C₆-C₁₀-Aryl, C₇-C₁₂-Aralkyl oder C₇-C₁₂-Alkylaryl bedeuten, wobei
- R²⁰, R²¹_{:}: gemeinsam auch eine Alkylenkette mit 2 bis 5 Kohlenstoffatomen, die durch N oder O unterbrochen sein kann, bedeuten können.

Im Hinblick auf die Formeln (IX), (X) und (XI) haben die Variablen insbesondere die folgende Bedeutung:
- R¹¹, R¹²:: stehen jeweils unabhängig voneinander für einen unverzweigten, verzweigten oder C₁- bis C₄-Alkyl Rest oder
- R¹¹ und R¹²: stehen gemeinsam oder für einen C₃- bis C₇-Alkandiyl-Rest, C₃- bis C₇-Alkendiyl-Rest, C₅- bis C₇-Cycloalkandiyl-Rest oder einen C₅- bis C₇-Cycloalkendiyl-Rest, wobei die vier vorgenannten Reste unsubstituiert sind oder einen oder mehrere gleiche oder verschiedene Substituenten tragen, die ausgewählt sind unter C₁- bis C₄-Alkyl;
- R¹³, R¹⁴:: jeweils unabhängig voneinander Wasserstoff oder geradkettiges oder verzweigtes C₁- bis C₄-Alkyl bedeuten und
- R¹⁵, R¹⁶, R¹⁷, R¹⁸:: für Phenyl stehen.

Im Rahmen des erfindungsgemäßen Verfahrens insbesondere bevorzugte chirale, zweizähnige Bisphosphin-Liganden sind solche der allgemeinen Formel (IX), insbesondere die im folgenden als "Chiraphos" bezeichnete Verbindungen der Formel (1) bzw. der Formeln (IXa) oder (IXb),

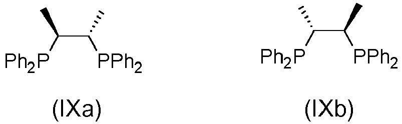

Geeignet sind auch die als "Norphos" bezeichnete Verbindung der Formel (5) bzw. der Formeln (IXc) oder (IXd),

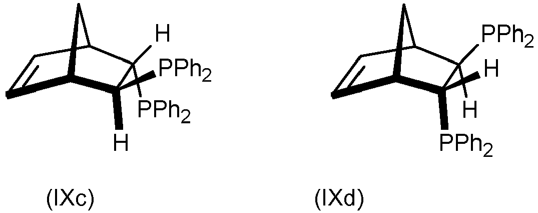

In den Formeln (IXa) bis (IXd) steht Ph für Phenyl.

Die gewählten chiralen Liganden werden erfindungsgemäß jeweils in Form eines ihrer beiden Enantiomeren eingesetzt. Die chiralen Liganden weisen typischerweise einen Enantiomerenüberschuss (ee) von wenigstens 80 %ee, insbesondere wenigstens 90 %ee und speziell wenigstens 95 %ee auf.

Bei Einsatz von chiralen Liganden mit zwei Phosphoratomen setzt man diese vorteilhaft in einer Menge von etwa 0,9 bis etwa 10 mol, bevorzugt etwa 1 bis etwa 4 mol pro mol an eingesetzter Übergangsmetall-Verbindung ein.

Bevorzugt wird der im Reaktionsgemisch lösliche, optisch aktive Rhodium-Katalysator durch Umsetzung einer achiralen Rhodiumverbindung und mit einem chiralen, zweizähnigen Bisphosphin-Liganden vor oder während der Hydrierung in-situ generiert.

In diesem Zusammenhang bedeutet der Ausdruck "in situ", dass der Katalysator direkt vor oder zu Beginn der Hydrierung generiert wird. Bevorzugt wird der Katalysator vor der Hydrierung generiert.

In einer bevorzugten Ausführungsform des erfindungsgenäßen Verfahrens wird der Katalysator vor der Hydrierung mit einem Kohlenmonoxid und Wasserstoff enthaltenden Gasgemisch vorbehandelt und/oder die asymmetrische Hydrierung in Gegenwart von dem Reaktionsgemisch zusätzlich zugeführtem Kohlenmonoxid durchführt.

Dies bedeutet, dass man den eingesetzten, im Reaktionsgemisch löslichen, d.h. homogenen Rhodium-Katalysator entweder vor der asymmetrischen Hydrierung mit einem Gasgemisch vorbehandelt, das Kohlenmonoxid und Wasserstoff enthält (d.h. eine sogenannte Präformierung durchführt) oder die asymmetrische Hydrierung in Gegenwart von dem Reaktionsgemisch zusätzlich zugeführtem Kohlenmonoxid durchführt oder eine Präformierung durchführt und anschließend die asymmetrische Hydrierung in Gegenwart von dem Reaktionsgemisch zusätzlich zugeführtem Kohlenmonoxid durchführt.

Bevorzugt wird Katalysator in dieser bevorzugten Ausführungsform mit einem Kohlenmonoxid und Wasserstoff enthaltendem Gasgemisch vorbehandelt und die asymmetrische Hydrierung in Gegenwart von dem Reaktionsgemisch zusätzlich zugeführtem Kohlenmonoxid durchführt.

Die in dieser bevorzugten Ausführungsform eingesetzten, im Reaktionsgemisch löslichen Rhodium-Katalysatoren können daher zumindest in einer im Katalysezyklus durchlaufenen Form oder in einer dem eigentlichen Katalysezyklus vorgeschalteten Vorform mindestens einen CO-Liganden aufweisen, wobei es unerheblich ist, ob diese mindestens einen CO-Liganden aufweisende Katalysatorform die tatsächliche katalytisch aktive Katalysatorform darstellt. Um die gegebenenfalls CO-Liganden aufweisende Katalysatorformen zu stabilisieren, kann es vorteilhaft sein dem Reaktionsgemisch währen der Hydrierung zusätzlich Kohlenmonoxid zuzuführen.

In dieser bevorzugten Ausführungsform wird die genannte Vorbehandlung der Katalysator-Vorstufe mit einem Gasgemisch umfassend 20 bis 90 Vol.-% Kohlenmonoxid, 10 bis 80 Vol-% Wasserstoff und 0 bis 5 Vol.-% weiterer Gase, wobei sich die genannten Volumenanteile zu 100 Vol.-% ergänzen, bei einem Druck von 5 bis 100 bar durchführt. Zusätzlich wird von dem so erhaltenen Katalysator vor Einsatz in der asymmetrischen Hydrierung überschüssiges Kohlenmonoxid abgetrennt.

Unter dem Begriff überschüssiges Kohlenmonoxid ist dabei solches Kohlenmonoxid zu verstehen, das in dem erhaltenen Reaktionsgemisch in gasförmiger oder gelöster Form enthalten ist und nicht an den Übergangsmetall-Katalysator bzw. dessen Vorstufe gebunden ist. Demgemäß entfernt man das überschüssige, nicht an den Katalysator gebundene Kohlenmonoxid zumindest weitgehend, d.h. in einem Ausmaß, dass sich eventuelle Restmengen gelösten Kohlenmonoxids in der folgenden Hydrierung nicht störend bemerkbar machen. Dies ist üblicherweise gewährleistet, wenn etwa 90%, bevorzugt etwa 95% oder mehr des zur Präformierung eingesetzten Kohlenmonoxids abgetrennt werden. Bevorzugt entfernt man das überschüssige Kohlenmonoxid vollständig von dem durch Präformierung erhaltenen Katalysator.

Die Abtrennung des überschüssigen Kohlenmonoxids vom erhaltenen Katalysator bzw. vom den Katalysator enthaltenden Reaktionsgemisch kann auf verschiedenen Wegen erfolgen. Bevorzugt entspannt man den Katalysator bzw. das durch Präformierung erhaltene, den Katalysator enthaltende Gemisch auf einen Druck von bis zu etwa 5 bar (absolut), vorzugsweise, speziell, bei Durchführung der Präformierung im Druckbereich von 5 bis 10 bar, auf einen Druck von weniger als 5 bar (absolut), bevorzugt auf einen Druck im Bereich von etwa 1 bar bis etwa 5 bar, vorzugsweise 1 bis weniger als 5 bar, besonders bevorzugt auf einen Druck im Bereich von 1 bis 3 bar, ganz besonders bevorzugt auf einen Druck im Bereich von etwa 1 bis etwa 2 bar, insbesondere bevorzugt auf Normaldruck, so dass gasförmiges, nicht gebundenes Kohlenmonoxid aus dem Produkt der Präformierung entweicht.

Die vorstehend genannte Entspannung des präformierten Katalysators kann beispielsweise unter Einsatz eines Hochdruckabscheiders, wie er dem Fachmann an sich bekannt ist, erfolgen. Derartige Abscheider, bei denen die Flüssigkeit in der kontinuierlichen Phase ist, sind beispielsweise beschrieben in: Perry's Chemical Engineers' Handbook, 1997, 7. Aufl., McGraw-Hill, S. 14.95 und 14.96; die Verhinderung eines möglichen Tropfenmitrisses ist auf den Seiten 14.87 bis 14.90 beschrieben. Die Entspannung des präformierten Katalysators kann einstufig oder zweistufig bis zum Erreichen des gewünschten Druckes im Bereich von 1 bar bis etwa 5 bar erfolgen, wobei die Temperatur üblicherweise auf 10 bis 40°C sinkt.

Alternativ kann die Abtrennung überschüssigen Kohlenmonoxids durch sogenanntes Strippen des Katalysators bzw. des den Katalysator enthaltenden Gemischs mit einem Gas, vorteilhaft mit einem unter den Reaktionsbedingungen inerten Gas, erreicht werden. Unter dem Begriff Strippen versteht der Fachmann dabei das Einleiten eines Gases in den Katalysator bzw. das den Katalysator enthaltende Reaktionsgemisch wie beispielsweise in W. R. A. Vauck, H. A. Müller, Grundoperationen chemischer Verfahrenstechnik, Deutscher Verlag für Grundstoffchemie Leipzig, Stuttgart, 10 Auflage, 1984, Seite 800, beschrieben. Als geeignete inerte Gase seien hierfür beispielhaft genannt: Wasserstoff, Helium, Neon, Argon, Xenon, Stickstoff und/oder CO₂, bevorzugt Wasserstoff, Stickstoff, Argon.

Bevorzugt wird die asymmetrische Hydrierung anschließend mit Wasserstoff durchführt, das einen Kohlenmonoxidgehalt im Bereich von 50 bis 3000 ppm, insbesondere im Bereich von 100 bis 2000 ppm, speziell im Bereich von 200 bis 1000 ppm und ganz speziell im Bereich von 400 bis 800 ppm aufweist.

Führt man eine Präformierung des Rhodium-Katalysators durch, löst man üblicherweise die gewählte Übergangsmetall-Verbindung und den gewählten chiralen Liganden und gewünschtenfalls das asymmetrisch zu hydrierende Substrat in einem geeigneten, unter den Reaktionsbedingungen inerten Lösungsmittel bzw. Lösungsmedium wie beispielsweise Ether, Tetrahydrofuran, Methyltetrahydrofuran, Toluol, Xylole, Chlorbenzol, Octadecanol, Biphenylether, Texanol, Marlotherm, Oxoöl9N (Hydroformylierungsprodukte aus isomeren Octenen, BASF Aktiengesellschaft), Citronellal und dergleichen. Als Lösungsmedium können auch das umzusetzende Substrat, das Produkt oder bei der Umsetzung eventuell anfallende hochsiedende Nebenprodukte dienen. Der resultierenden Lösung wird, vorteilhafterweise in einem geeigneten Druckreaktor bzw. Autoklaven, bei einem Druck von üblicherweise etwa 5 bis etwa 350 bar, bevorzugt von etwa 20 bis etwa 200 bar und besonders bevorzugt von etwa 50 bis etwa 100 bar ein Gasgemisch aufgepresst, das Wasserstoff und Kohlenmonoxid enthält. Bevorzugt setzt man zur Präformierung ein Gasgemisch ein, das etwa
30 bis 99 Vol.-% Wasserstoff,
1 bis 70 Vol.-% Kohlenmonoxid und
0 bis 5 Vol.-% weiterer Gase enthält,
wobei sich die Angaben in Vol.-% zu 100 Vol.-% addieren müssen.

Besonders bevorzugt setzt man zur Präformierung ein Gasgemisch ein, das etwa
40 bis 80 Vol.-% Wasserstoff,
20 bis 60 Vol.-% Kohlenmonoxid und
0 bis 5 Vol.-% weiterer Gase enthält,
wobei sich die Angaben in Vol.-% zu 100 Vol.-% addieren müssen.

Ein zur Präformierung insbesondere bevorzugtes Gasgemisch ist sogenanntes Synthesegas, das üblicherweise zu etwa 35 bis 55 Vol-% aus Kohlenmonoxid neben Wasserstoff und Spuren weiterer Gase besteht.

Die Präformierung des Katalysators wird üblicherweise bei Temperaturen von etwa 25°C bis etwa 100°C, bevorzugt bei etwa 40°C bis etwa 80°C durchgeführt. Führt man die Präformierung in Gegenwart des asymmetrisch zu hydrierenden Substrats durch, wählt man die Temperatur mit Vorteil so, dass es nicht in störendem Ausmaß zu einer Isomerisierung der zu hydrierenden Doppelbindung kommt. Die Präformierung ist üblicherweise nach etwa 1 h bis etwa 24 h, oft nach etwa 1 bis etwa 12 h abgeschlossen. Im Anschluss an die optional durchzuführende Präformierung führt man erfindungsgemäß die asymmetrische Hydrierung des gewählten Substrats durch. Nach vorangegangener Präformierung lässt sich das gewählte Substrat in der Regel mit gutem Erfolg mit oder ohne Zuführung von zusätzlichem Kohlenmonoxid durchführen. Wird keine Präformierung durchgeführt, kann die erfindungsgemäße asymmetrische Hydrierung entweder in Gegenwart von dem Reaktionssystem zugeführtem Kohlenmonoxid oder ohne die Zuführung von Kohlenmonoxid durchgeführt werden. Vorteilhafterweise führt man eine Präformierung wie beschrieben durch und setzt dem Reaktionsgemisch während der asymmetrischen Hydrierung zusätzliches Kohlenmonoxid zu.

Falls dem Reaktionssystem Kohlenmonoxid zugeführt wird, kann die Zuführungauf verschiedene Weise vorgenommen werden: So kann das Kohlenmonoxid beispielsweise dem zur asymmetrischen Hydrierung eingesetzten Wasserstoff beigemischt werden oder auch direkt gasförmig in die Reaktionslösung eindosiert werden. Eine weitere Möglichkeit besteht beispielsweise darin, dem Reaktionsgemisch Verbindungen zuzusetzen, die leicht Kohlenmonoxid freisetzen, wie beispielsweise Formiate oder Oxalyl-Verbindungen.

Bevorzugt wird das Kohlenmonoxid dem zur asymmetrischen Hydrierung eingesetzten Wasserstoff beigemischt.

Die erfindungsgemäße asymmetrische Hydrierung nimmt man vorteilhaft bei einem Druck von etwa 2 bis etwa 200 bar, insbesondere von etwa 10 bis etwa 100 bar, speziell bei etwa 60 bis etwa 100 bar und einer Temperatur von in der Regel etwa 0°C bis etwa 100°C, bevorzugt etwa 0°C bis etwa 30°C, insbesondere bei etwa 10°C bis etwa 30°C vor.

Die Wahl des zur Durchführung der erfindungsgemäßen asymmetrischen Hydrierung einzusetzenden Lösemittels ist von geringerer Bedeutung. In jedem Fall treten erfindungsgemäßen Vorteile auch in unterschiedlichen Lösungsmitteln auf. Geeignete Lösemittel sind beispielsweise jene zur Durchführung der erfindungsgemäßen Präformierung genannten. Mit besonderem Vorteil führt man die asymmetrische Hydrierung im gleichen Lösemittel durch wie die gegebenenfalls zuvor durchgeführte Präformierung.

Als Reaktionsgefäße zur Durchführung der erfindungsgemäßen asymmetrischen Hydrierung sind prinzipiell all jene geeignet, die Umsetzungen unter den genannten Bedingungen, insbesondere Druck und Temperatur, erlauben und für Hydrierreaktionen geeignet sind, wie beispielsweise Autoklaven, Rohrreaktoren, Blasensäulen, etc.

Führt man die Hydrierung des erfindungsgemäßen Verfahrens unter Einsatz von hochsiedenden, in der Regel viskosen Lösungsmitteln durch, wie sie beispielsweise vorstehend zum Einsatz im Rahmen der Vorbehandlung des Katalysators beschrieben sind (etwa die genannten Lösungsmittel Octadecanol, Biphenylether, Texanol, Marlotherm®, Oxoöl9N) oder führt man die Hydrierung ohne zusätzlichen Einsatz von Lösemittel jedoch unter Aufpegelung der als Nebenprodukte in geringem Ausmaß entstehenden Hochsieder (wie beispielsweise Dimere oder Trimere, die durch Reaktionen der Edukte bzw. Produkte und anschließenden Folgereaktionen entstehen) durch, kann es vorteilhaft sein, für guten Gaseintrag und gute Durchmischung von Gasphase und kondensierter Phase zu sorgen. Dies gelingt beispielsweise dadurch, dass man den Hydrierschritt des erfindungsgemäßen Verfahrens in einem Gasumlaufreaktor durchführt. Gasumlaufreaktoren sind dem Fachmann an sich bekannt und beispielsweise in P. Trambouze, J.-P. Euzen, Chemical Reactors, Ed. Technip, 2004, S. 280-283 und P. Zehner, R. Benfer, Chem. Eng. Sci. 1996, 51, 1735-1744 sowie z. B. in der EP 1 140 349 beschrieben. Grundsätzlich ist es auch möglich, die Umsetzung in dem Produkt als Lösungsmittel durchzuführen, beispielsweise in Citronellal, wenn es sich bei dem Edukt um Neral oder Geranial handelt.

Bei Einsatz eines wie vorstehend genannten Gasumlaufreaktors hat es sich als besonders vorteilhaft erwiesen, das einzusetzende Gas bzw. Gasgemisch (den Kohlenmonoxid enthaltenden Wasserstoff) parallel zu den in den Reaktor eingetragenen Edukten und/oder dem umlaufenden Reaktionsgemische bzw. dem Katalysator mittels einer einfachen Düse oder einer Zweistoffdüse in den Gasumlaufreaktor einzutragen. Dabei zeichnet sich die Zweistoffdüse dadurch aus, dass in den Reaktor einzubringende Flüssigkeit und Gas durch zwei getrennte ineinander liegenden Röhren unter Druck bis zum Düsenmund gelangen und dort miteinander vereint werden.

Das erfindungsgemäße Verfahren kann mit gutem Erfolg mit und ohne Zugabe von tertiären Aminen durchgeführt werden. Bevorzugt führt man das erfindungsgemäße Verfahren in Abwesenheit, d.h. ohne Zugabe von zusätzlichen tertiären Aminen oder in Anwesenheit nur katalytischer Mengen von zusätzlichen tertiären Aminen durch. Die verwendete Aminmenge kann dabei zwischen 0,5 und 500 mol-Äquivalenten bezogen auf die eingesetzte Menge an Metall, bevorzugt aber 1 bis 100 mol-Äquivalenten bezogen auf die eingesetzte Metallmenge betragen. Die Wahl des tertiären Amins ist nicht kritisch. Neben kurzkettigen Alkylaminen, wie beispielsweise Triethylamin können auch langkettige Alkylamine, wie zum Beispiel Tridodecylamin, verwendet werden. Im Rahmen einer bevorzugten Ausführungsform führt man das erfindungsgemäße Hydrierverfahren in Gegenwart eines tertiären Amins, bevorzugt Tridodecylamin, in einer Menge von etwa 2 bis 30 mol-Äquivalenten, bevorzugt etwa 5 bis 20 mol-Äquivalenten und besonders bevorzugt 5 bis 15 mol-Äquivalenten bezogen auf die Menge an eingesetztem Übergangsmetall ein.

Mit Vorteil bricht man die Reaktion ab, wenn die Zielverbindung in der gewünschten Ausbeute und der gewünschte optischen Aktivität, d.h. mit dem gewünschten Enantiomerenüberschuss (ee) im Reaktionsgemisch vorliegt, wie es der Fachmann durch Routineuntersuchungen beispielsweise mittels chromatographischer Methoden feststellen kann. Üblicherweise ist die Hydrierung nach etwa 1 bis etwa 150 h, oft nach etwa 2 bis etwa 24 h abgeschlossen.

Durch das erfindungsgemäße Verfahren gelingt es, optisch aktive Carbonylverbindungen, insbesondere optisch aktive Aldehyde in hohen Ausbeuten und Enantiomerenüberschüssen bereitzustellen. Üblicherweise erhält man die gewünschten asymmetrisch hydrierten Verbindungen in einem Enantiomerenüberschuss von mindestens 80 % ee, oft mit einem Enantiomerenüberschuss von etwa 85 bis etwa 99 % ee. Dabei ist zu beachten, dass der maximal erzielbare Enantiomerenüberschuss von der Reinheit des eingesetzten Substrats, insbesondere im Hinblick auf die Isomerenreinheit der zu hydrierenden Doppelbindung, abhängen kann.

Demzufolge eignen sich als Ausgangssubstanzen insbesondere solche, die ein Isomerenverhältnis von mindestens etwa 90:10, bevorzugt mindestens etwa 95:5 bezüglich der E/Z-Doppelbindungsisomere aufweisen.

Durch die Präformierung und/oder durch das zusätzlich ins Reaktionssystem eingebrachte Kohlenmonoxid können die eingesetzten homogenen Katalysatoren stabilisiert werden, wodurch zum einen die Standzeit der Katalysatoren deutlich erhöht wird und zum anderen die Wiederverwendbarkeit der homogenen Katalysatoren ermöglicht wird.

So lässt sich beispielsweise das erhaltene Reaktionsprodukt durch dem Fachmann an sich bekannte Verfahren, wie z.B. durch Destillation, aus dem Reaktionsgemisch entfernen und der zurückbleibende Katalysator, gegebenenfalls nach abermaliger Präformierung, im Rahmen weiterer Umsetzungen nutzen.

Das erfindungsgemäße Verfahren kann demgemäß sowohl diskontinuierlich bzw. semikontinuierlich als auch kontinuierlich betrieben werden und eignet sich insbesondere für Umsetzungen in technischem Maßstab.

Im Rahmen einer besonders bevorzugten Ausführungsform des erfindungsgemäßen Verfahrens setzt man Neral oder Geranial, das bis zu etwa 5 mol-%, bevorzugt bis zu etwa 2 mol-% des jeweiligen Doppelbindungsisomeren enthält, zu optisch aktivem Citronellal um. Zur Bildung des Katalysators setzt man bevorzugt eine im Reaktionsgemisch lösliche Verbindung des Rhodiums, insbesondere Rh(OAc)₃, [Rh(cod)Cl]₂, Rh(CO)₂acac, [Rh(cod)OH]₂, [Rh(cod)OMe]₂, Rh₄(CO)₁₂, Rh₂(OAc)₄, Bis(ethylen)rhodium(I) acetylacetonat oder Rh₆(CO)₁₆ und als chiralen Liganden (R,R)-Chiraphos bzw. (S,S)-Chiraphos ((2R, 3R)-(+)-2,3-bis(Diphenylphosphino)butan bzw. (2S, 3S)-(-)-2,3-bis(Diphenylphosphino)butan) im molaren Verhältnis von etwa 1:1 bis etwa 1:4 ein, wobei das Reaktionsgemisch während der Hydrierung von Neral oder Geranial zusätzlich wenigstens eine Verbindung der allgemeinen Formel (I) enthält, die insbesondere ausgewählt ist unter einer der vorgenannten Verbindungen (I-1) bis (I-12) und speziell unter (*R,R*)-Chiraphosmonooxid, (*S,S*)-Chiraphosmonooxid, Cyclohexyldiphenylphosphin, Cyclopentyldiphenylphosphin, 2-Butyldiphenylphosphin oder Isopropyldiphenylphosphin, wobei man die Verbindung der Formel (I) insbesondere in einer Menge von vorzugsweise 0,03 bis 0,6 Mol pro Mol Rhodium einsetzt.

In einer insbesondere bevorzugten Ausführungsform des erfindungsgemäßen Verfahren setzt man Neral, das bis zu etwa 5 mol-%, bevorzugt bis zu etwa 2 mol-% Geranial enthält, in Gegenwart einer im Reaktionsgemisch löslichen Verbindung des Rhodiums, wie Rh(OAc)₃, [Rh(cod)Cl]₂, Rh(CO)₂acac, [Rh(cod)OH]₂, [Rh(cod)OMe]₂, Rh₄(CO)₁₂, Rh₂(OAc)₄, Bis(ethylen)rhodium(I) acetylacetonat oder Rh₆(CO)₁₆ und (R,R)-Chiraphos zum D-Citronellal um, wobei das Reaktionsgemisch während der Hydrierung zusätzlich wenigstens eine Verbindung der allgemeinen Formel (I) enthält, die insbesondere ausgewählt ist unter einer der vorgenannten Verbindungen (I-1) bis (I-12) und speziell unter (*R,R*)-Chiraphosmonooxid, (*S,S*)-Chiraphosmonooxid, Cyclohexyldiphenylphosphin, Cyclopentyldiphenylphosphin, 2-Butyldiphenylphosphin oder Isopropyldiphenylphosphin, wobei man die Verbindung der Formel (I) insbesondere in einer Menge von 0,03 bis 0,6 Mol pro Mol Rhodium einsetzt. In einer anderen, ebenfalls besonders bevorzugten Ausführungsform des erfindungsgemäßen Verfahren setzt man Neral, das bis zu etwa 5 mol-%, bevorzugt bis zu etwa 2 mol-% Geranial enthält, in Gegenwart einer im Reaktionsgemisch löslichen Verbindung des Rhodiums, wie Rh(OAc)₃, [Rh(cod)Cl]₂, Rh(CO)₂acac, [Rh(cod)OH]₂, [Rh(cod)OMe]₂, Rh₄(CO)₁₂, Rh₂(OAc)₄, Bis(ethylen)rhodium(I) acetylacetonat oder Rh₆(CO)₁₆ und (S,S)-Chiraphos zum L-Citronellal um, wobei das Reaktionsgemisch während der Hydrierung zusätzlich wenigstens eine Verbindung der allgemeinen Formel (I) enthält, die insbesondere ausgewählt ist unter einer der vorgenannten Verbindungen (I-1) bis (I-12) und speziell unter (*R,R*)-Chiraphosmonooxid, (*S,S*)-Chiraphosmonooxid, Cyclohexyldiphenylphosphin, Cyclopentyldiphenylphosphin, 2-Butyldiphenylphosphin oder Isopropyldiphenylphosphin, wobei man die Verbindung der Formel (I) insbesondere in einer Menge von 0,03 bis 0,6 Mol pro Mol Rhodium einsetzt. Bevorzugt präformiert man den Katalysator unter den vorstehend genannten Bedingungen und führt anschließend die asymmetrische Hydrierung in Gegenwart von Wasserstoff durch, der insbesondere 50 bis 3000 ppm Kohlenmonoxid enthält. Im Rahmen der bevorzugten Ausführungsform verzichtet man vorteilhaft auf den Zusatz von Lösemitteln und führt die genannten Umsetzungen im umzusetzenden Substrat bzw. dem Produkt und gegebenenfalls in hochsiedenden Nebenprodukten als Lösemedium durch. Insbesondere bevorzugt ist die kontinuierliche Reaktionsführung unter Wiederverwendung bzw. Rückführung des erfindungsgemäß stabilisierten homogenen Katalysators.

Grundsätzlich kann man (R,R)-Chiraphos durch (R,R)-Norphos ((2R,3R)-2,3-Bis(diphenylphosphino)bicyclo[2.2.1]hept-4-en) bzw. (S,S)-Chiraphos durch (S,S)-Norphos ((2S,3S)-2,3-Bis(diphenylphosphino)bicyclo[2.2.1]hept-4-en) ersetzen.

Ein weiterer Aspekt der vorliegenden Erfindung betrifft ein Verfahren zur Herstellung von optisch aktivem Menthol unter Verwendung von nach dem erfindungsgemäßen Verfahren hergestellten optisch aktiven Citronellal. Die Herstellung von optisch aktivem Menthol ausgehend von optisch aktivem Citronellal ist bekannt. Ein Schlüsselschritt ist hierbei die Cyclisierung von optisch aktivem Citronellal zu optisch aktivem Isopulegol wie beispielsweise in der EP-A 1 225 163 beschrieben.

Das Verfahren zur Herstellung von optisch aktivem Menthol umfasst die folgenden Schritte:
i) Herstellung von optisch aktivem Citronellal durch asymmetrische Hydrierung von Geranial der Formel (IIa-1) oder von Neral der Formel (IIb-1) nach dem erfindungsgemäßen Verfahren,
ii) Cyclisierung des so hergestellten optisch aktiven Citronellals zu optisch aktivem Isopulegol in Gegenwart einer Lewis-Säure und
iii) Hydrierung des so hergestellten optisch aktiven Isopulegols zu optisch aktivem Menthol.

Das erfindungsgemäß hergestellte optisch aktive Citronellal lässt sich, wie nachstehend schematisch für die Herstellung von L-Menthol der Formel (XIII) dargestellt, in Gegenwart einer geeigneten Säure, insbesondere einer Lewis-Säure zum L-Isopulegol der Formel (XII) cyclisieren und anschließend zum L-Menthol hydrieren.

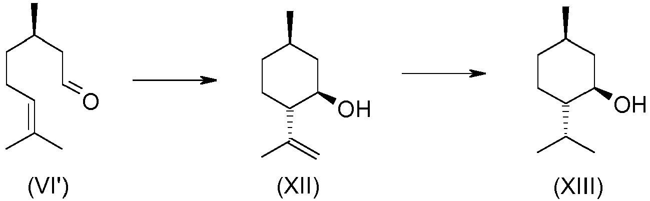

Ein weiterer Aspekt der vorliegenden Erfindung betrifft demgemäß die Verwendung von nach dem erfindungsgemäßen Verfahren hergestellten optisch aktivem Citronellal zur Herstellung von optisch aktivem Menthol. Insbesondere betrifft die Erfindung die Verwendung des nach dem erfindungsgemäßen Verfahren hergestellten D-Citronellals zur Herstellung von optisch aktivem L-Menthol.

Die folgenden Beispiele dienen der Verdeutlichung der Erfindung, ohne sie in irgendeiner Weise zu beeinträchtigen:

### BEISPIELE

### Vergleichsbeispiel 1: Asymmetrische Hydrierung von Neral in Gegenwart von Kohlenmonoxid

Rh(CO)₂acac (43 mg, 0,17 mmol) und (R,R)-Chiraphos (105 mg, 0,24 mmol) wurden in Texanol (28 ml, 2,2,4-Trimethyl-1,3-pentandiol monoisobutyrat, Sigma-Aldrich) gelöst und in einem 100 ml Stahlautoklaven (V2A-Stahl, Hersteller Premex, magnetgekoppelter Begasungsrührer, 1000 Umdrehungen/min) bei 80 bar Synthesegas (H₂/CO = 1:1, Vol./Vol.) und 70°C für 16 h gerührt. Anschließend wird auf 25°C gekühlt und für zwei Stunden Stickstoff durch die Lösung geleitet (8 l/h). Nach der Spülung mit Stickstoff wurde Neral (16,2 g, Verhältnis der Doppelbindungsisomere Neral/Geranial = 98,3:0,4; molares Verhältnis Substrat/Katalysator = 650) über eine Schleuse in den Autoklaven zugegeben. Der Reaktionsdruck wurde durch Zupressen von Wasserstoffgas, das 1000 ppm Kohlenmonoxid enthielt, auf 80 bar eingestellt. Zeit/Umsatz wurde gaschromatographisch bestimmt. Nach 4 Stunden Reaktionszeit wurde ein Umsatz von 39% erreicht und eine Ausbeute von 39% an Citronellal. Nach 20 h lag der Umsatz an Citral bei >99% und die Ausbeute an D-Citronellal ebenfalls bei >99% wobei die optische Reinheit mit 85% ee bestimmt wurde.

### Beispiele 2a - 2o: Asymmetrische Hydrierung von Neral in Gegenwart von Kohlenmonoxid unter Zugabe eines Promotors (Tabelle 1) bei c(Rh) = 400 ppm

Rh(CO)₂acac (43 mg, 0,17 mmol), (R,R)-Chiraphos (105 mg, 0,24 mmol) und ein Additiv (siehe Tabelle 1) wurden in Texanol (28 ml, 2,2,4-Trimethyl-1,3-pentandiol monoisobutyrat, Sigma-Aldrich) gelöst und in einem 100 ml Stahlautoklaven (V2A-Stahl, Hersteller Premex, magnetgekoppelter Begasungsrührer, 1000 Umdrehungen/min) bei 80 bar Synthesegas (H₂/CO = 1:1, Vol./Vol.) und 70°C für 16 h gerührt. Anschließend wird auf 25°C gekühlt und für zwei Stunden Stickstoff durch die Lösung geleitet (8 l/h). Nach der Spülung mit Stickstoff wurden 16,2 g Neral (Verhältnis der Doppelbindungsisomere Neral/Geranial = 98,3:0,4; Verhältnis Substrat/Katalysator = 650) über eine Schleuse in den Autoklaven zugegeben. Der Reaktionsdruck wurde durch Zupressen von Wasserstoffgas, das 1000 ppm Kohlenmonoxid enthielt, auf 80 bar eingestellt. Zeit/Umsatz wurde gaschromatographisch bestimmt. Umsätze/Enantioselektivitäten/Additive sind in Tabelle 1 angegeben.

### Beispiel 2p-q: Asymmetrische Hydrierung von Neral in Gegenwart von Kohlenmonoxid unter Zugabe eines Promotors (Tabelle 1) bei c(Rh) = 700 ppm

Rh(CO)₂acac (75 mg, 0,29 mmol), (R,R)-Chiraphos (186 mg, 0,44 mmol) und ein Additiv (siehe Tabelle 1) wurden in Texanol (28 ml, 2,2,4-Trimethyl-1,3-pentandiol monoisobutyrat, Sigma-Aldrich) gelöst und in einem 100 ml Stahlautoklaven (V2A-Stahl, Hersteller Premex, magnetgekoppelter Begasungsrührer, 1000 Umdrehungen/min) bei 80 bar Synthesegas (H₂/CO = 1:1, Vol./Vol.) und 70°C für 16 h gerührt. Anschließend wird auf 25°C gekühlt und für zwei Stunden Stickstoff durch die Lösung geleitet (8 l/h). Nach der Spülung mit Stickstoff wurden 16,2 g Neral (Verhältnis der Doppelbindungsisomere Neral/Geranial = 98,3:0,4; Verhältnis Substrat/Katalysator = 365) über eine Schleuse in den Autoklaven zugegeben. Der Reaktionsdruck wurde durch Zupressen von Wasserstoffgas, das 1000 ppm Kohlenmonoxid enthielt, auf 80 bar eingestellt. Zeit/Umsatz wurde gaschromatographisch bestimmt. Umsätze/Enantioselektivitäten/Additive sind in Tabelle 1 angegeben.

### Vergleichsbeispiele 2r-s: Asymmetrische Hydrierung von Neral in Gegenwart von Kohlenmonoxid bei Zugabe des Promotors ohne bidentaten Liganden (Tabelle 1) bei c(Rh) = 400 ppm

Rh(CO)₂acac (43 mg, 0,17 mmol) und der Phosphin-Ligand (0,17 mmol, siehe Tabelle 1) wurden in Texanol (28 ml, 2,2,4-Trimethyl-1,3-pentandiol monoisobutyrat, Sigma-Aldrich) gelöst und in einem 100 ml Stahlautoklaven (V2A-Stahl, Hersteller Premex, magnetgekoppelter Begasungsrührer, 1000 Umdrehungen/min) bei 80 bar Synthesegas (H₂/CO = 1:1, Vol./Vol.) und 70°C für 16 h gerührt. Anschließend wird auf 25°C gekühlt und für zwei Stunden Stickstoff durch die Lösung geleitet (8 l/h). Nach der Spülung mit Stickstoff wurden 16,2 g Neral (Verhältnis der Doppelbindungsisomere Neral/Geranial = 98,3:0,4; Verhältnis Substrat/Katalysator = 650) über eine Schleuse in den Autoklaven zugegeben. Der Reaktionsdruck wurde durch Zupressen von Wasserstoffgas, das 1000 ppm Kohlenmonoxid enthielt, auf 80 bar eingestellt. Zeit/Umsatz wurde gaschromatographisch bestimmt. Umsätze und Enantioselektivitäten sind in Tabelle 1 angegeben.

### Vergleichsbeispiel 2t

Die Umsetzung wurden nach der für Beispiel 2b beschriebenen Vorschrift durchgeführt, wobei das Lösungsmittel Texanol durch die gleiche Menge D-Citronellal (88 % ee) ersetzt wurde und dem Reaktionsgemisch kein (R,R)-Chiraphosmonooxid als Additiv zugesetzt wurde. Umsätze und Enantioselektivitäten sind in Tabelle 1 angegeben.

### Beispiel 2u:

Die Umsetzung wurden nach der für Beispiel 2b beschriebenen Vorschrift durchgeführt, wobei das Lösungsmittel Texanol durch die gleiche Menge D-Citronellal (88 % ee) ersetzt wurde. Umsätze und Enantioselektivitäten sind in Tabelle 1 angegeben.

### Vergleichsbeispiel 2v

Die Umsetzung wurden nach der für Beispiel 2b beschriebenen Vorschrift durchgeführt, wobei (R,R)-Chiraphos wurde durch die gleiche Menge (R,R)-Norphos ersetzt wurde und dem Reaktionsgemisch kein (R,R)-Chiraphosmonooxid als Additiv zugesetzt wurde. Umsätze und Enantioselektivitäten sind in Tabelle 1 angegeben.

### Beispiel 2w:

Die Umsetzung wurden nach der für Beispiel 2b beschriebenen Vorschrift durchgeführt, wobei (R,R)-Chiraphos wurde durch die gleiche Menge (R,R)-Norphos ersetzt wurde. Umsätze und Enantioselektivitäten sind in Tabelle 1 angegeben.

### Beispiel 2x:

Die Umsetzung wurden nach der für Beispiel 2b beschriebenen Vorschrift durchgeführt, wobei anstelle des Kohlenmonoxid-haltigen Wasserstoff reiner Wasserstoff eingesetzt wurde. Umsätze und Enantioselektivitäten sind in Tabelle 1 angegeben.

### Vergleichsbeispiel 2y

Die Umsetzung wurden nach der für Beispiel 2a beschriebenen Vorschrift durchgeführt, wobei dem Reaktionsgemisch 1,05 mmol Tridodecylamin als Additiv zugesetzt wurde. Umsätze und Enantioselektivitäten sind in Tabelle 1 angegeben.

### Beispiel 2z:

Die Umsetzung wurden nach der für Beispiel 2y beschriebenen Vorschrift durchgeführt, wobei dem Reaktionsgemisch zusätzlich zum Tridodecylamin (R,R)-Chiraphosmonooxid als Additiv zugesetzt wurde. Umsätze und Enantioselektivitäten sind in Tabelle 1 angegeben.

### Beispiel 3: Herstellung von (R,R)-Chiraphosmonooxid (CPMO) durch Oxidation von (R,R)-Chiraphos mit Wasserstoffperoxid

(R,R)-Chiraphos (230 g, 0,54 mol) wird in einem 5 L-Doppelmantelrührgefäß bei 25°C unter Rühren in Toluol (3500 mL) gelöst und während 8 Stunden über eine Spritzempumpe kontinuierlich mit Wasserstoffperoxid (15 %ig in Wasser, 9 g, 0.26 mol) versetzt. Die Temperatur wird bei 25°C gehalten (die durch Exothermie verursachte Temperaturerhöhung ist minimal). Die Reaktion wird noch über Nacht für weitere 10 Stunden bei 25°C gerührt. Der Reaktionsaustrag wird am Rotationsverdampfer bei 150 mbar und 75°C bis auf 900 ml eingeengt (klare gelbliche Lösung). 100ml der eingeengten Lösung werden bei 75°C/5 mbar vollständig eingeengt und in der Glovebox über eine 10*10 cm Kieselgelsäule aufgereinigt. Zur Abtrennung von nicht umgesetztem Chiraphos wird zunächst mit Toluol eluiert (1500 mL), danach mit Methanol (600 mL), Kontrolle der Fraktionen mittels GC (Säule: 30m/0,32mm HP-5; Injektor: 280°C, Detektor: FID; 320°C, Programm: 100°C → 20°C/Min → 300°C). Es werden 3,1 g einer Mischung aus CPMO:CPDO = 76:24 (auf Basis ³¹P-NMR) erhalten. ³¹P-NMR (C₆D₆, 500 MHz): 33.5 ppm (CPDO), 33.0 ppm (d, 30 Hz), -8.1 ppm (d, 30 Hz)

**Tabelle 1: Übersicht der Ergebnisse der Beispiele 1 und 2a-s**

| **Beispiel** | **Verbindung (I)^{a)} [mmol]** | **mol% bezo gen auf Chi raphos** | **c (Rh, gesamt) [ppm]** | **4 h [%] ^{e)}** | **20 h [%] ^{f)}** | **%ee** |
|---|---|---|---|---|---|---|
| 1*) | - | - | 400 | 39 | >99 | 85 |
| 2a | CPMO^{b)} (0,066) | 26% | 400 | 93 | >99 | 87 |
| 2b | CPMO^{b)} (0,075) | 30% | 400 | 97 | >99 | 87 |
| 2c*) | TPP (0,028) | 10% | 400 | 45 | 98 | 85 |
| 2d*) | TPP (0,081) | 30% | 400 | 44 | >99 | 87 |
| 2e | CyDPP (0,026) | 10% | 400 | 68 | >99 | 85 |
| 2f | CyDPP (0,077) | 30% | 400 | 98 | >99 | 87 |
| 2g | iPrDPP (0,028) | 10% | 400 | 57 | 97 | 84 |
| 2h | iPrDPP (0,076) | 30% | 400 | 95 | >99 | 87 |
| 2i*⁾ | HexDPP (0,025) | 10% | 400 | 34 | 99 | 83 |
| 2j*⁾ | HexDPP (0,076) | 30% | 400 | 33 | >99 | 87 |
| 2k | CPMO^{b)} (0,024) | 10% | 400 | 55 | >99 | 87 |
| 2l | CPMO^{b)} (0,034) | 15% | 400 | 84 | >99 | 87 |
| 2m*⁾ | DPPE-O | 10% | 400 | 28 | 98 | 83 |
| 2n*⁾ | DPPE-O | 30% | 400 | 26 | 98 | 85 |
| 2o*⁾ | CPDO | 30% | 400 | 23 | >99 | 84 |
| 2p*⁾ | - | - | 700 | 75 | >99 | 87 |
| 2q | CPMO^{b)} (0,130) | 30% | 700 | >99 | >99 | 87 |
| 2r*⁾ | nur CPMO^{b)} | CMPO:Rh = 1:1 | 400 | 1 | 1 | n.b.^{d)} |
| 2s*⁾ | nur iPrDPP | iPrDPP:Rh = 1:1 | 400 | 4 | 6 | n.b.^{d)} |
| 2t*^{) g)} | - | - | 400 | 68 | >99 | 89 |
| 2u^{g)} | CPMO^{b)} (0,075) | 30 % | 400 | 79 | >99 | 89 |
| 2v*^{) h)} | - | - | 400 | 14 | 70 | 83 |
| 2w^{h)} | CPMO^{b)} (0,075) | 30 % | 400 | 41 | 95 | 84 |
| 2xⁱ⁾ | CPMO^{b)} (0,075) | 30 % | 400 | 88 | 98 | 87 |
| 2y*⁾ | Tridodecylamin (1,05) | 400 % | 400 | 54 | 99 | 86 |
| 2z | Tridodecylamin (1,05) | 400 % | 400 | 97 | 99 | 86 |
| | CPMO^{b)} (0,075) | 30 % | | | | |

| | | | | | | |
|---|---|---|---|---|---|---|
| *) Bei den mit einem Stern markierten Beispielen handelt es sich um Vergleichsbeispiele. a) siehe Tabelle 2; b) CPMO wurde Zugegeben als Mischung von 75% CPMO mit 23% CPDO (Herstellung entsprechend Beispiel 3); die zugegebene Menge bezieht sich auf die zugegebene Menge reinen CPMOs in dieser Mischung; d) nicht bestimmt, zu geringer Umsatz; e) Umsatz nach 4h; f) Umsatz nach 20 h; g) Das Lösungsmittel Texanol wurde durch D-Citronellal (88 % ee) ersetzt; h) (R,R)-Chiraphos wurde durch (R,R)-Norphos ersetzt; i) Zugeführter Wasserstoff enthält kein CO. | | | | | | |

**Tabelle 2: Ligandenstrukturen**

| | | | |
|---|---|---|---|
| 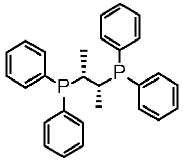 | 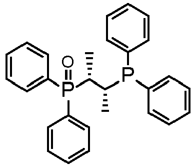 | 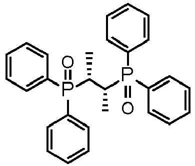 | 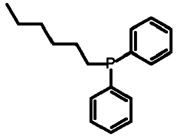 |
| (*R,R*)-Chiraphos (CAS 74839-84-2) | (*R,R*)-Chiraphosmonooxid (CPMO, keine CAS vergeben) | (*R,R*)-Chiraphosdioxid (CPDO, CAS 192449-15-3) | Hexyl-Diphenylphosphin (HexylDPP, CAS 18298-00-5) |
| 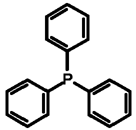 | 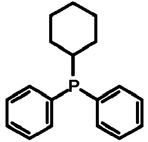 | 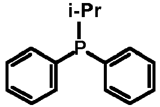 | 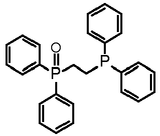 |
| Triphenylphosphin (TPP, CAS 603-35-0) | Cyclohexyldiohenylphosphin (CyDPP, CAS 6372-42-5) | Isopropyldiphenylphosphin (iPrDPP, CAS 6372-40-3) | 1,2-Bis(diphenylphosphin)-ethanmonooxid (DPPE-O, CAS 984-43-0, Sigma-Aldrich) |
| 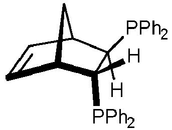 | | | |
| (R, R)-Norphos CAS 71042-55-2- | | | |

## Patentansprüche

1. Verfahren zur Herstellung einer optisch aktiven Carbonylverbindung durch asymmetrische Hydrierung einer prochiralen α,β-ungesättigter Carbonylverbindung mit Wasserstoff in Gegenwart wenigstens eines im Reaktionsgemisch löslichen, optisch aktiven Übergangsmetall-Katalysators, der Rhodium als katalytisch aktives Übergangsmetall und einen chiralen, zweizähnigen Bisphosphin-Liganden aufweist, wobei das Reaktionsgemisch während der Hydrierung der prochiralen α,β-ungesättigter Carbonylverbindung zusätzlich wenigstens eine Verbindung der allgemeinen Formel (I) enthält: 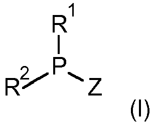 worin
R¹, R²: gleich oder verschieden sind und für C₆- bis C₁₀-Aryl stehen, das unsubstituiert ist oder einen oder mehrere Substituenten trägt, die ausgewählt sind unter C₁- bis C₆-Alkyl, C₃- bis C₆-Cycloalkyl, C₆- bis C₁₀-Aryl, C₁- bis C₆-Alkoxy und Amino;
Z für eine Gruppe CHR³R⁴ oder Aryl steht, das unsubstituiert ist oder einen oder mehrere Substituenten trägt, die ausgewählt sind unter C₁- bis C₆-Alkyl, C₃- bis C₆-Cycloalkyl, C₆- bis C₁₀-Aryl, C₁- bis C₆-Alkoxy und Amino, worin
R³ für C₁- bis C₄-Alkyl, C₁- bis C₄-Alkoxy-C₁- bis C₄-alkyl, C₃- bis C₆-Cycloalkyl oder C₆- bis C₁₀-Aryl steht, wobei eine oder zwei nicht benachbarte CH₂-Gruppen in C₃- bis C₆-Cycloalkyl auch durch ein Sauerstoffatom ersetzt sein können;
R⁴ für C₁- bis C₄-Alkyl, das unsubstituiert ist oder eine Gruppe P(=O)R^{4a}R^{4b} trägt, C₁- bis C₄-Alkoxy, C₁- bis C₄-Alkoxy-C₁- bis C₄-alkyl, C₃- bis C₆-Cycloalkyl oder C₆- bis C₁₀-Aryl steht, wobei eine oder zwei nicht benachbarte CH₂-Gruppen in C₃- bis C₆-Cycloalkyl auch durch ein Sauerstoffatom ersetzt sein können und wobei C₃- bis C₆-Cycloalkyl und C₆- bis C₁₀-Aryl unsubstituiert sind oder einen oder mehrere Substituenten tragen, die ausgewählt sind unter C₁- bis C₄-Alkyl, C₁- bis C₄-Alkoxy und Amino;
oder
R³, R⁴: zusammen mit dem C-Atom, an das sie gebunden sind, für C₄- bis C₈-Cycloalkyl steht, worin eine oder zwei nicht benachbarte CH₂-Gruppen in C₃- bis C₆-Cycloalkyl auch durch ein Sauerstoffatom ersetzt sein können und wobei C₃- bis C₆-Cycloalkyl unsubstituiert ist oder einen oder mehrere Subsitutenten trägt, die ausgewählt sind unter C₁- bis C₄-Alkyl, C₁- bis C₄-Alkoxy und A-P(=O)R^{4a}R^{4b},
wobei A für eine chemische Bindung oder für C₁- bis C₄-Alkylen steht; und
R^{4a}, R^{4b}: gleich oder verschieden sind und für Phenyl stehen, das unsubstituiert ist oder einen oder mehrere Substituenten trägt, die ausgewählt sind unter C₁- bis C₆-Alkyl, C₃- bis C₆-Cycloalkyl, C₆- bis C₁₀-Aryl, C₁- bis C₆-Alkoxy und Amino.

2. Verfahren nach Anspruch 1, wobei Z in Formel (I) für eine Gruppe CHR³R⁴ steht.

3. Verfahren nach Anspruch 2, wobei die Variablen R¹, R², Z in Formel (I) die folgenden Bedeutungen aufweisen:
R¹, R²: gleich oder verschieden sind und für Phenyl stehen, das unsubstituiert ist oder 1, 2 oder 3 Substituenten trägt, die ausgewählt sind unter Methyl und Methoxy;
Z für eine Gruppe CHR³R⁴ steht, worin
R³ für C₁- bis C₄-Alkyl steht;
R⁴ für C₁- bis C₄-Alkyl, das unsubstituiert ist oder eine Gruppe P(=O)R^{4a}R^{4b} trägt;
oder
R³, R⁴: zusammen mit dem C-Atom, an das sie gebunden sind, für C₃- bis C₈-Cycloalkyl steht, worin eine oder zwei CH₂-Gruppen durch ein bzw. zwei Sauerstoffatome ersetzt sein können und wobei C₃- bis C₆-Cycloalkyl unsubstituiert ist oder eine Gruppe A-P(=O)R^{4a}R^{4b} trägt und/oder 1, 2, 3 oder 4 Methylgruppen als Substituenten aufweist;
wobei A für eine chemische Bindung, CH₂ oder CH(CH₃) steht; und
R^{4a}, R^{4b}: gleich oder verschieden sind und für C₆- bis C₁₀-Aryl stehen, das unsubstituiert ist oder 1, 2 oder 3 Substituenten trägt, die ausgewählt sind unter Methyl und Methoxy.

4. Verfahren nach Anspruch 2, worin
R¹, R²: für unsubstituiertes Phenyl stehen;
R³ für Methyl steht;
R⁴ eine Gruppe CH(CH₃)-P(=O)R^{4a}R^{4b} bedeutet, worin R^{4a} und R^{4b} jeweils für unsubstituiertes Phenyl stehen.

5. Verfahren nach einem der vorhergehenden Ansprüche, wobei man die Verbindung der Formel (I) in einer Menge von 0,001 Mol bis 1 Mol pro Mol Rhodium einsetzt.

6. Verfahren nach einem der vorhergehenden Ansprüche, wobei die prochirale α,β-ungesättigte Carbonylverbindung ein prochirales, α,β-ungesättigtes Keton oder ein prochiraler, α,β-ungesättigter Aldehyd ist.

7. Verfahren nach einem der vorhergehenden Ansprüche, wobei die prochirale α,β-ungesättigte Carbonylverbindung ausgewählt ist unter Verbindungen der allgemeinen Formel (II) 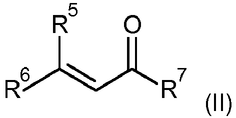 worin
R⁵, R⁶ voneinander verschieden sind und jeweils für einen unverzweigten, verzweigten oder cyclischen Kohlenwassestoffrest mit 1 bis 25 Kohlenstoffatomen steht, der gesättigt ist oder eine oder mehrere, nicht konjugierte ethylenische Doppelbindungen aufweist, und der unsubstituiert ist oder einen oder mehrere gleiche oder verschiedene Substituenten trägt, die ausgewählt sind unter OR⁸, NR^{9a}R^{9b}, Halogen, C₆- bis C₁₀-Aryl und C₃- bis C₉-Hetaryl;
R⁷ für Wasserstoff oder für einen unverzweigten, verzweigten oder cyclischen Kohlenwassestoffrest mit 1 bis 25 Kohlenstoffatomen steht, der gesättigt ist oder eine oder mehrere, nicht konjugierte ethylenische Doppelbindungen aufweist, und der unsubstituiert ist oder einen oder mehrere gleiche oder verschiedene Substituenten trägt, die ausgewählt sind unter OR⁸, NR^{9a}R^{9b}, Halogen, C₆- bis C₁₀-Aryl und C₃- bis C₉-Hetaryl;
oder
R⁷ gemeinsam mit einem der Reste R⁵ oder R⁶ auch eine 3 bis 25-gliedrige Alkylengruppe bedeuten kann worin 1, 2, 3 oder 4 nicht benachbarte CH₂-Gruppen durch O oder N-R^{9c} ersetzt sein können, wobei die Alkylengruppe gesättigt ist oder eine oder mehrere, nicht konjugierte ethylenische Doppelbindungen aufweist, und wobei die Alkylengruppe unsubstituiert ist oder einen oder mehrere gleiche oder verschiedene Substituenten trägt, die ausgewählt sind unter OR⁸, NR^{9a}R^{9b}, Halogen, C₁- bis C₄-Alkyl, C₆- bis C₁₀-Aryl und C₃- bis C₉-Hetaryl, wobei zwei Substituenten auch gemeinsam für eine 2 bis 10-gliedrigrige Alkylengruppe stehen können, wobei die 2- bis 10-gliedrige Alkylengruppe gesättigt ist oder eine oder mehrere, nicht konjugierte ethylenische Doppelbindungen aufweist, und wobei die 2- bis 10-gliedrige Alkylengruppe unsubstituiert ist oder einen oder mehrere gleiche oder verschiedene Substituenten trägt, die ausgewählt sind unter OR⁸, NR^{9a}R^{9b}, Halogen, C₆- bis C₁₀-Aryl und C₃- bis C₉-Hetaryl;
wobei
R⁸ für Wasserstoff, C₁- bis C₆-Alkyl, C₆- bis C₁₀-Aryl, C₇- bis C₁₂-Aralkyl oder C₇- bis C₁₂-Alkylaryl steht;
R^{9a}, R^{9b} jeweils unabhängig voneinander Wasserstoff, C₁- bis C₆-Alkyl, C₆- bis C₁₀-Aryl, C₇- bis C₁₂-Aralkyl oder C₇- bis C₁₂-Alkylaryl bedeuten oder
R^{9a} und R^{9b} gemeinsam auch eine Alkylenkette mit 2 bis 5 Kohlenstoffatomen, die durch N oder O unterbrochen sein kann, bedeuten können; und
R^{9c} für Wasserstoff, C₁- bis C₆-Alkyl, C₆- bis C₁₀-Aryl, C₇- bis C₁₂-Aralkyl oder C₇- bis C₁₂-Alkylaryl steht.

8. Verfahren nach einem der vorhergehenden Ansprüche, wobei die prochirale α,β-ungesättigte Carbonylverbindung ausgewählt ist unter Verbindungen der allgemeinen Formel (IIa) und (IIb) 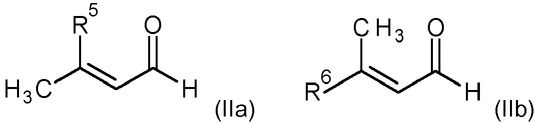 worin
R⁵, R⁶ jeweils für einen unverzweigten oder verzweigten Kohlenwassestoffrest mit 2 bis 25 Kohlenstoffatomen steht, der gesättigt ist oder 1, 2, 3, 4 oder 5 nicht konjugierte ethylenische Doppelbindungen aufweist;

9. Verfahren nach Anspruch 8 zur Herstellung von optisch aktivem Citronellal der Formel (IV) 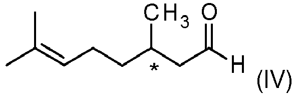 worin * das Asymmetriezentrum bezeichnet;
durch asymmetrische Hydrierung von Geranial der Formel (IIa-1) oder von Neral der Formel (IIb-1)

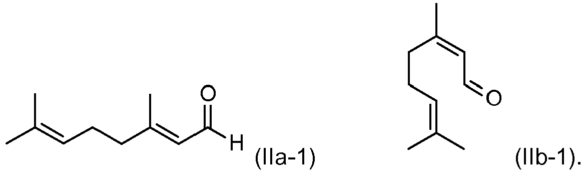

10. Verfahren nach Anspruch 9 zur Herstellung von D-Citronellal durch asymmetrische Hydrierung von Neral.

11. Verfahren nach einem der vorhergehenden Ansprüche, wobei der chirale, zweizähnige Bisphosphin-Ligand eine Verbindung der allgemeinen Formeln (IX), (X) oder (XI) ist: 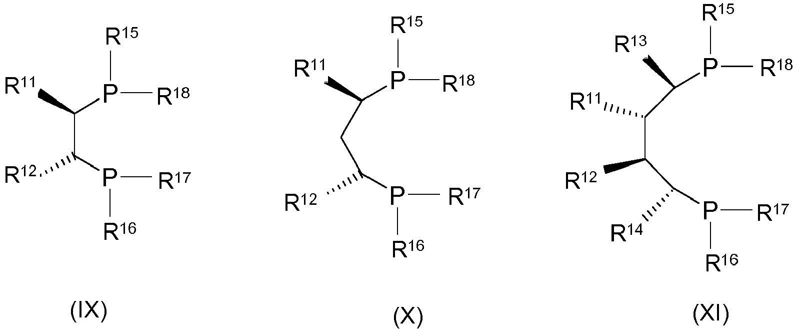 worin
R¹¹, R¹²: jeweils unabhängig voneinander für einen unverzweigten, verzweigten oder cyclischen Kohlenwasserstoffrest mit 1 bis 20 Kohlenstoffatomen stehen, der gesättigt ist oder eine oder mehrere, in der Regel 1 bis etwa 4, nicht konjugierte, ethylenische Doppelbindungen aufweisen kann und der unsubstituiert ist oder einen oder mehrere, in der Regel 1 bis 4, gleiche oder verschiedene Substituenten trägt, die ausgewählt sind unter OR¹⁹, NR²⁰R²¹, Halogen, C₆-C₁₀-Aryl und C₃-C₉-Hetaryl, oder
R¹¹ und R¹² gemeinsam auch eine 2 bis 10-gliedrige Alkylengruppe oder eine 3 bis 10-gliedrige Cycloalkylengruppe bedeuten kann worin 1, 2, 3 oder 4 nicht benachbarte CH₂-Gruppen durch O oder N-R^{9c} ersetzt sein können, wobei die Alkylengruppe und die Cycloalkylengruppe gesättigt sind oder eine oder zwei, nicht konjugierte ethylenische Doppelbindungen aufweisen, und wobei die Alkylengruppe und die Cycloalkylengruppe unsubstituiert sind oder einen oder mehrere gleiche oder verschiedene Substituenten tragen, die ausgewählt sind unter C₁- bis C₄-Alkyl;
R¹³, R¹⁴: jeweils unabhängig voneinander Wasserstoff oder geradkettiges oder verzweigtes C₁- bis C₄-Alkyl bedeuten und
R¹⁵, R¹⁶, R¹⁷, R¹⁸: gleich oder verschieden sind und für C₆- bis C₁₀-Aryl stehen, das unsubstituiert ist oder einen oder mehrere Substituenten trägt, die ausgewählt sind unter C₁- bis C₆-Alkyl, C₃- bis C₆-Cycloalkyl, C₆- bis C₁₀-Aryl, C₁- bis C₆-Alkoxy und Amino;
R¹⁹, R²⁰, R²¹: jeweils unabhängig voneinander Wasserstoff, C₁C₄-Alkyl, C₆-C₁₀-Aryl, C₇-C₁₂-Aralkyl oder C₇-C₁₂-Alkylaryl bedeuten, wobei
R²⁰, R²¹: gemeinsam auch eine Alkylenkette mit 2 bis 5 Kohlenstoffatomen, die durch N oder O unterbrochen sein kann, bedeuten können.

12. Verfahren nach Anspruch 11, wobei der chirale, zweizähnige Bisphosphin-Ligand eine Verbindung der allgemeinen Formel (IX) ist.

13. Verfahren nach Anspruch 12, wobei der chirale, zweizähnige Bisphosphin-Ligand entweder die Verbindung der Formel (IXa) oder der Formel (IXb) ist: 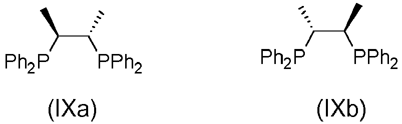 worin Ph für Phenyl steht.

14. Verfahren nach Anspruch 12, wobei der chirale, zweizähnige Bisphosphin-Ligand entweder die Verbindung der Formel (IXc) oder oder der Formel (IXd) ist: 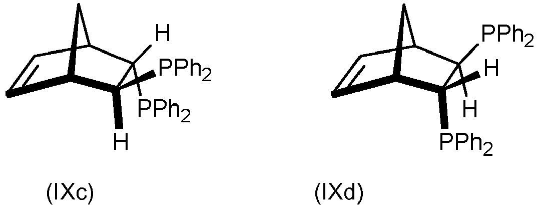 worin Ph für Phenyl steht.

15. Verfahren nach einem der vorhergehenden Ansprüche, das wenigstens eines oder alle der folgenden Merkmale a - g aufweist:
a) Durchführung der asymmetrische Hydrierung bei einem Wasserstoff-Druck von 5 bis 200 bar, insbesondere bei einem Wasserstoff-Druck von 10 bis 100 bar;
b) Kontinuierliche Durchführung des Verfahrens;
c) in-situ Generierung des Katalysators vor oder während der Hydrierung durch Umsetzung einer achiralen Rhodiumverbindung und mit einem chiralen, zweizähnigen Bisphosphin-Liganden;
d) Vorbehandlung des Katalysators vor der Hydrierung mit einem Kohlenmonoxid und Wasserstoff enthaltenden Gasgemisch;
e) Durchführung der asymmetrische Hydrierung in Gegenwart von dem Reaktionsgemisch zusätzlich zugeführtem Kohlenmonoxid;
f) Durchführung der asymmetrischen Hydrierung mit Wasserstoff, das einen Kohlenmonoxidgehalt im Bereich von 50 bis 3000 ppm, insbesondere im Bereich von 100 bis 2000 ppm, speziell im Bereich von 200 bis 1000 ppm und ganz speziell im Bereich von 400 bis 800 ppm aufweist;
g) Durchführung der asymmetrischen Hydrierung in einem Gasumlaufreaktor;
h) Durchführung der asymmetrischen Hydrierung in einem Gasumlaufreaktor, wobei man die umzusetzende α,β-ungesättigte Carbonylverbindung und den Wasserstoff mittels einer Zweistoffdüse in den Gasumlaufreaktor einträgt.

16. Verfahren zur Herstellung von optisch aktivem Menthol umfassend die Schritte
i) Herstellung von optisch aktivem Citronellal durch asymmetrische Hydrierung gemäß Anspruch 9 von Geranial der Formel (IIa-1) oder von Neral der Formel (IIb-1),
ii) Cyclisierung des so hergestellten optisch aktiven Citronellals zu optisch aktivem Isopulegol in Gegenwart einer Lewis-Säure und
iii) Hydrierung des so hergestellten optisch aktiven Isopulegols zu optisch aktivem Menthol.

## Claims

1. A process for the preparation of an optically active carbonyl compound by asymmetric hydrogenation of a prochiral α,β-unsaturated carbonyl compound with hydrogen in the presence of at least one optically active transition metal catalyst that is soluble in the reaction mixture and which has rhodium as catalytically active transition metal and a chiral, bidentate bisphosphine ligand, wherein the reaction mixture during the hydrogenation of the prochiral α,β-unsaturated carbonyl compound additionally comprises at least one compound of the general formula (I) : 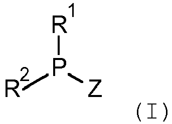 in which
R¹, R²: are identical or different and are C₆- to C₁₀-aryl which is unsubstituted or carries one or more substituents which are selected from C₁- to C₆-alkyl, C₃- to C₆-cycloalkyl, C₆- to C₁₀-aryl, C₁- to C₆-alkoxy and amino;
Z is a group CHR³R⁴ or aryl which is unsubstituted or carries one or more substituents which are selected from C₁- to C₆-alkyl, C₃- to C₆-cycloalkyl, C₆- to C₁₀-aryl, C₁- to C₆-alkoxy and amino, in which
R³ is C₁- to C₄-alkyl, C₁- to C₄-alkoxy-C₁- to C₄-alkyl, C₃- to C₆-cycloalkyl or C₆- to C₁₀-aryl, where one or two nonadjacent CH₂ groups in C₃- to C₆-cycloalkyl can also be replaced by an oxygen atom;
R⁴ is C₁- to C₄-alkyl which is unsubstituted or carries a group P(=O)R^{4a}R^{4b,} C₁- to C₄-alkoxy, C₁- to C₄-alkoxy-C₁- to C₄-alkyl, C₃- to C₆-cycloalkyl or C₆- to C₁₀-aryl, where one or two nonadjacent CH₂ groups in C₃- to C₆-cycloalkyl can also be replaced by an oxygen atom, and where C₃- to C₆-cycloalkyl and C₆- to C₁₀-aryl are unsubstituted or carry one or more substituents which are selected from C₁- to C₄-alkyl, C₁- to C₄-alkoxy and amino;
or
R³, R⁴: together with the carbon atom to which they are bonded, are C₄- to C₈-cycloalkyl, in which one or two nonadjacent CH₂ groups in C₃- to C₆-cycloalkyl can also be replaced by an oxygen atom and where C₃- to C₆-cycloalkyl is unsubstituted or carries one or more substituents which are selected from C₁- to C₄-alkyl, C₁- to C₄-alkoxy and A-P(=O)R^{4a}R^{4b},
where A is a chemical bond or a C₁- to C₄-alkylene;
and
R^{4a}, R^{4b}: are identical or different and are phenyl which is unsubstituted or carries one or more substituents which are selected from C₁- to C₆-alkyl, C₃- to C₆-cycloalkyl, C₆- to C₁₀-aryl, C₁- to C₆-alkoxy and amino.

2. The process according to claim 1, wherein Z in formula (I) is a group CHR³R⁴.

3. The process according to claim 2, wherein the variables R¹, R², Z in formula (I) have the following meanings:
R¹, R²: are identical or different and are phenyl which is unsubstituted or carries 1, 2 or 3 substituents which are selected from methyl and methoxy;
Z is a group CHR³R⁴, in which
R³ is C₁- to C₄-alkyl;
R⁴ is C₁- to C₄-alkyl which is unsubstituted or carries a group P (=O)R^{4a}R^{4b};
or
R³, R⁴: together with the carbon atom to which they are bonded, is C₃- to C₈-cycloalkyl in which one or two CH₂ groups can be replaced by one or two oxygen atoms and where C₃- to C₆-cycloalkyl is unsubstituted or carries a group A-P(=O)R^{4a}R^{4b} and/or has 1, 2, 3 or 4 methyl groups as substituents;
where A is a chemical bond, CH₂ or CH(CH₃) ; and
R^{4a}, R^{4b}: are identical or different and are C₆- to C₁₀-aryl which is unsubstituted or carries 1, 2 or 3 substituents which are selected from methyl and methoxy.

4. The process according to claim 2, in which
R¹, R²: are unsubstituted phenyl;
R³ is methyl;
R⁴ is a group CH(CH₃) -P (=O)R^{4a}R^{4b} in which R^{4a} and R^{4b} are in each case unsubstituted phenyl.

5. The process according to one of the preceding claims, wherein the compound of the formula (I) is used in an amount of from 0.001 mol to 1 mol per mole of rhodium.

6. The process according to one of the preceding claims, wherein the prochiral α,β-unsaturated carbonyl compound is a prochiral, α,β-unsaturated ketone or a prochiral, α,β-unsaturated aldehyde.

7. The process according to one of the preceding claims, wherein the prochiral α,β-unsaturated carbonyl compound is selected from compounds of the general formula (II) 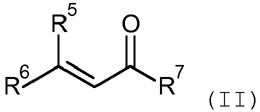 in which
R⁵, R⁶ are different from one another and is in each case an unbranched, branched or cyclic hydrocarbon radical having 1 to 25 carbon atoms which is saturated or has one or more, nonconjugated ethylenic double bonds, and which is unsubstituted or carries one or more identical or different substituents which are selected from OR⁸, NR^{9a}R^{9b}, halogen, C₆- to C₁₀-aryl and C₃- to C₉-hetaryl;
R⁷ is hydrogen or an unbranched, branched or cyclic hydrocarbon radical having 1 to 25 carbon atoms which is saturated or has one or more nonconjugated ethylenic double bonds, and which is unsubstituted or carries one or more identical or different substituents which are selected from OR⁸, NR^{9a}R^{9b}, halogen, C₆- to C₁₀-aryl and C₃- to C₉-hetaryl;
or
R⁷ together with one of the radicals R⁵ or R⁶, can also be a 3- to 25-membered alkylene group, in which 1, 2, 3 or 4 nonadjacent CH₂ groups can be replaced by O or N-R^{9c}, where the alkylene group is saturated or has one or more nonconjugated ethylenic double bonds, and where the alkylene group is unsubstituted or carries or one or more identical or different substituents which are selected from OR⁸, NR^{9a}R^{9b}, halogen, C₁- to C₄-alkyl, C₆- to C₁₀-aryl and C₃- to C₉-hetaryl, where two substituents also together can be a 2- to 10-membered alkylene group, where the 2- to 10-membered alkylene group is saturated or has one or more nonconjugated ethylenic double bonds, and where the 2- to 10-membered alkylene group is unsubstituted or carries one or more identical or different substituents which are selected from OR⁸, NR^{9a}R^{9b}, halogen, C₆- to C₁₀-aryl and C₃- to C₉-hetaryl;
where
R⁸ is hydrogen, C₁- to C₆-alkyl, C₆- to C₁₀-aryl, C₇- to C₁₂-aralkyl or C₇- to C₁₂-alkylaryl;
R^{9a}, R^{9b} are in each case independently of one another hydrogen, C₁- to C₆-alkyl, C₆- to C₁₀-aryl, C₇- to C₁₂-aralkyl or C₇- to C₁₂-alkylaryl or
R^{9a} and R^{9b} together can also be an alkylene chain having 2 to 5 carbon atoms, which can be interrupted by N or O; and
R^{9c} is hydrogen, C₁- to C₆-alkyl, C₆- to C₁₀-aryl, C₇- to C₁₂-aralkyl or C₇- to C₁₂-alkylaryl.

8. The process according to one of the preceding claims, wherein the prochiral α,β-unsaturated carbonyl compound is selected from compounds of the general formula (IIa) and (IIb) 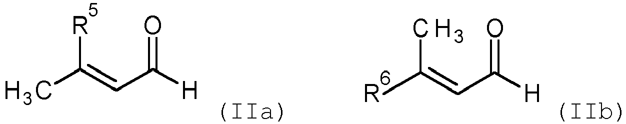 in which
R⁵, R⁶ is in each case an unbranched or branched hydrocarbon radical having 2 to 25 carbon atoms which is saturated or has 1, 2, 3, 4 or 5 nonconjugated ethylenic double bonds.

9. The process according to claim 8 for the preparation of optically active citronellal of the formula (IV) 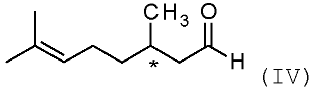 in which * designates the asymmetry center;
by asymmetric hydrogenation of geranial of the formula (IIa-1) or of neral of the formula (IIb-1)

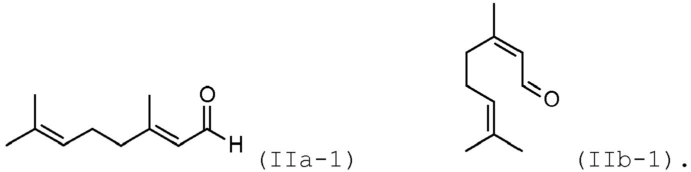

10. The process according to claim 9 for producing D-citronellal by asymmetric hydrogenation of neral.

11. The process according to one of the preceding claims, wherein the chiral, bidentate bisphosphine ligand is a compound of the general formulae (IX), (X) or (XI): 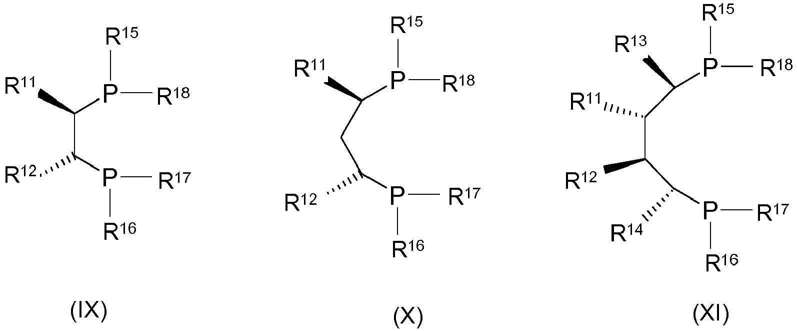 in which
R¹¹, R¹²: in each case independently of one another are an unbranched, branched or cyclic hydrocarbon radical having 1 to 20 carbon atoms which is saturated or can have one or more, generally 1 to about 4, nonconjugated, ethylenic double bonds and is unsubstituted or carries one or more, generally 1 to 4, identical or different substituents which are selected from OR¹⁹, NR²⁰R²¹, halogen, C₆-C₁₀-aryl and C₃-C₉-hetaryl, or
R¹¹ and R¹² together can also be a 2 to 10-membered alkylene group or a 3- to 10-membered cycloalkylene group, in which 1, 2, 3 or 4 nonadjacent CH₂ groups can be replaced by O or N-R^{9c}, where the alkylene group and the cycloalkylene group are saturated or have one or two nonconjugated ethylenic double bonds, and where the alkylene group and the cycloalkylene group are unsubstituted or carry one or more identical or different substituents which are selected from C₁- to C₄-alkyl;
R¹³, R¹⁴: are in each case independently of one another hydrogen or straight-chain or branched C₁- to C₄-alkyl and
R¹⁵, R¹⁶, R¹⁷, R¹⁸: are identical or different and are C₆- to C₁₀-aryl which is unsubstituted or carries one or more substituents which are selected from C₁- to C₆-alkyl, C₃- to C₆-cycloalkyl, C₆- to C₁₀-aryl, C₁- to C₆-alkoxy and amino;
R¹⁹, R²⁰, R²¹: are in each case independently of one another hydrogen, C₁-C₄-alkyl, C₆-C₁₀-aryl, C₇-C₁₂-aralkyl or C₇-C₁₂-alkylaryl, where
R²⁰, R²¹: can together also be an alkylene chain having 2 to 5 carbon atoms, which can be interrupted by N or O.

12. The process according to claim 11, wherein the chiral, bidentate bisphosphine ligand is a compound of the general formula (IX).

13. The process according to claim 12, wherein the chiral, bidentate bisphosphine ligand is either the compound of the formula (IXa) or the formula (IXb): 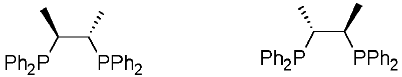 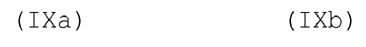 in which Ph is phenyl.

14. The process according to claim 12, wherein the chiral, bidentate bisphosphine ligand is either the compound of the formula (IXc) or the formula (IXd): 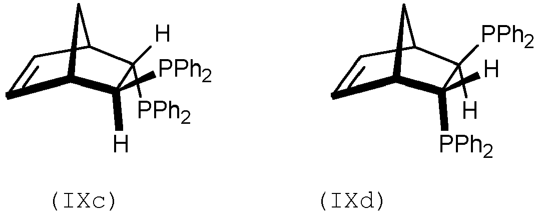 in which Ph is phenyl.

15. The process according to one of the preceding claims, which has at least one or all of the following features a - g:
a) implementation of the asymmetric hydrogenation at a hydrogen pressure of from 5 to 200 bar, in particular at a hydrogen pressure of from 10 to 100 bar;
b) continuous implementation of the process;
c) in-situ generation of the catalyst before or during the hydrogenation by reaction of an achiral rhodium compound and with a chiral, bidentate bisphosphine ligand;
d) pretreatment of the catalyst before the hydrogenation with a gas mixture comprising carbon monoxide and hydrogen;
e) implementation of the asymmetric hydrogenation in the presence of carbon monoxide additionally added to the reaction mixture;
f) implementation of the asymmetric hydrogenation with hydrogen which has a carbon monoxide content in the range from 50 to 3000 ppm, in particular in the range from 100 to 2000 ppm, specifically in the range from 200 to 1000 ppm and very specifically in the range from 400 to 800 ppm;
g) implementation of the asymmetric hydrogenation in a gas recirculation reactor;
h) implementation of the asymmetric hydrogenation in a gas recirculation reactor, where the α,β-unsaturated carbonyl compound to be reacted and the hydrogen are introduced into the gas recirculation reactor using a two-component nozzle.

16. A process for the preparation of optically active menthol, comprising the steps
i. preparation of optically active citronellal by asymmetric hydrogenation according to claim 9 from geranial of the formula (IIa-1) or from neral of the formula (IIb-1),
ii. cyclization of the optically active citronellal prepared in this way to give optically active isopulegol in the presence of a Lewis acid and
iii. hydrogenation of the thus prepared optically active isopulegol to give optically active menthol.

## Revendications

1. Procédé de fabrication d'un composé de carbonyle optiquement actif par hydrogénation asymétrique d'un composé de carbonyle α,β-insaturé prochiral avec de l'hydrogène en présence d'au moins un catalyseur de métal de transition optiquement actif soluble dans le mélange réactionnel, qui comprend du rhodium en tant que métal de transition catalytiquement actif et un ligand bisphosphine bidentate chiral, le mélange réactionnel contenant en outre pendant l'hydrogénation du composé de carbonyle α,β-insaturé prochiral au moins un composé de formule générale (I) : 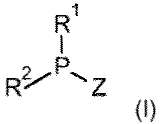 dans laquelle
R¹, R² : sont identiques ou différents, et représentent un aryle en C₆ à C₁₀, qui est non substitué ou porte un ou plusieurs substituants, qui sont choisis parmi alkyle en C₁ à C₆, cycloalkyle en C₃ à C₆, aryle en C₆ à C₁₀, alcoxy en C₁ à C₆ et amino ;
Z représente un groupe CHR³R⁴ ou un aryle, qui est non substitué ou porte un ou plusieurs substituants, qui sont choisis parmi alkyle en C₁ à C₆, cycloalkyle en C₃ à C₆, aryle en C₆ à C₁₀, alcoxy en C₁ à C₆ et amino ;
R³ représentant un alkyle en C₁ à C₄, un alcoxy en C₁ à C₄-alkyle en C₁ à C₄, un cycloalkyle en C₃ à C₆ ou un aryle en C₆ à C₁₀, un ou deux groupes CH₂ non voisins dans le cycloalkyle en C₃ à C₆ pouvant également être remplacés par un atome d'oxygène ;
R⁴ représentant un alkyle en C₁ à C₄, qui est non substitué ou porte un groupe P(=O)R^{4a}R^{4b}, un alcoxy en C₁ à C₄, un alcoxy en C₁ à C₄-alkyle en C₁ à C₄, un cycloalkyle en C₃ à C₆ ou un aryle en C₆ à C₁₀, un ou deux groupes CH₂ non voisins dans le cycloalkyle en C₃ à C₆ pouvant également être remplacés par un atome d'oxygène ; et le cycloalkyle en C₃ à C₆ et l'aryle en C₆ à C₁₀ étant non substitués ou portant un ou plusieurs substituants, qui sont choisis parmi alkyle en C₁ à C₄, alcoxy en C₁ à C₄ et amino ;
ou
R³, R⁴ : représentant ensemble avec l'atome C auquel ils sont reliés un cycloalkyle en C₄ à C₈, un ou deux groupes CH₂ non voisins dans le cycloalkyle en C₃ à C₆ pouvant également être remplacés par un atome d'oxygène ; et le cycloalkyle en C₃ à C₆ étant non substitué ou portant un ou plusieurs substituants, qui sont choisis parmi alkyle en C₁ à C₄, alcoxy en C₁ à C₄ et A-P(=O)R^{4a}R^{4b},
A représentant une liaison chimique ou un alkylène en C₁ à C₄ ;
et
R^{4a}, R^{4b} : étant identiques ou différents, et représentant un phényle, qui est non substitué ou porte un ou plusieurs substituants, qui sont choisis parmi alkyle en C₁ à C₆, cycloalkyle en C₃ à C₆, aryle en C₆ à C₁₀, alcoxy en C₁ à C₆ et amino.

2. Procédé selon la revendication 1, dans lequel Z dans la formule (I) représente un groupe CHR³R⁴.

3. Procédé selon la revendication 2, dans lequel les variables R¹, R², Z dans la formule (I) ont les significations suivantes :
R¹, R² : sont identiques ou différents, et représentent un phényle, qui est non substitué ou porte 1, 2 ou 3 substituants, qui sont choisis parmi méthyle et méthoxy ;
Z représente un groupe CHR³R⁴,
R³ représentant un alkyle en C₁ à C₄ ;
R⁴ représentant un alkyle en C₁ à C₄, qui est non substitué ou porte un groupe P(=O)R^{4a}R^{4b} ;
ou
R³, R⁴ : représentant ensemble avec l'atome C auquel ils sont reliés un cycloalkyle en C₃ à C₈, dans lequel un ou deux groupes CH₂ peuvent être remplacés par un ou deux atomes d'oxygène, le cycloalkyle en C₃ à C₆ étant non substitué ou portant un groupe A-P(=O)R^{4a}R^{4b} et/ou 1, 2, 3 ou 4 groupes méthyle en tant que substituants ;
A représentant une liaison chimique, CH₂ ou CH(CH₃) ;
et
R^{4a}, R^{4b} : étant identiques ou différents, et représentant un aryle en C₆ à C₁₀, qui est non substitué ou porte 1, 2 ou 3 substituants, qui sont choisis parmi méthyle et méthoxy.

4. Procédé selon la revendication 2, dans lequel
R¹, R² : représentant un phényle non substitué ;
R³ représente un méthyle ;
R⁴ signifie un groupe CH(CH₃)-P(=O)R^{4a}R^{4b}, R^{4a} et R^{4b} représentant chacun un phényle non substitué.

5. Procédé selon l'une quelconque des revendications précédentes, dans lequel le composé de formule (I) est utilisé en une quantité de 0,001 mole à 1 mole par mole de rhodium.

6. Procédé selon l'une quelconque des revendications précédentes, dans lequel le composé de carbonyle α,β-insaturé prochiral est une cétone α,β-insaturée prochirale ou un aldéhyde α,β-insaturé prochiral.

7. Procédé selon l'une quelconque des revendications précédentes, dans lequel le composé de carbonyle α,β-insaturé prochiral est choisi parmi les composés de formule générale (II) 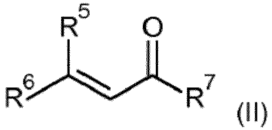 dans laquelle
R⁵, R⁶ sont différents l'un de l'autre et représentent chacun un radical hydrocarboné non ramifié, ramifié ou cyclique de 1 à 25 atomes de carbone, qui est saturé ou comprend une ou plusieurs doubles liaisons éthyléniques non conjuguées, et qui est non substitué ou porte un ou plusieurs substituants identiques ou différents, qui sont choisis parmi OR⁸, NR^{9a}R^{9b}, halogène, aryle en C₆ à C₁₀ et hétaryle en C₃ à C₉ ;
R⁷ représente l'hydrogène ou un radical hydrocarboné non ramifié, ramifié ou cyclique de 1 à 25 atomes de carbone, qui est saturé ou comprend une ou plusieurs doubles liaisons éthyléniques non conjuguées, et qui est non substitué ou porte un ou plusieurs substituants identiques ou différents, qui sont choisis parmi OR⁸, NR^{9a}R^{9b}, halogène, aryle en C₆ à C₁₀ et hétaryle en C₃ à C₉ ; ou
R⁷ peut également signifier ensemble avec un des radicaux R⁵ ou R⁶ un groupe alkylène de 3 à 25 chaînons, dans lequel 1, 2, 3 ou 4 groupes CH₂ non voisins peuvent être remplacés par O ou N-R^{9c}, le groupe alkylène étant saturé ou comprenant une ou plusieurs doubles liaisons éthyléniques non conjugués, et le groupe alkylène étant non substitué ou portant un ou plusieurs substituants identiques ou différents, qui sont choisis parmi OR⁸, NR^{9a}R^{9b}, halogène, alkyle en C₁ à C₄, aryle en C₆ à C₁₀ et hétaryle en C₃ à C₉ ; deux substituants pouvant également représenter ensemble un groupe alkylène de 2 à 10 chaînons, le groupe alkylène de 2 à 10 chaînons étant saturé ou comprenant une ou plusieurs doubles liaisons éthyléniques non conjuguées, et le groupe alkylène de 2 à 10 chaînons étant non substitué ou portant un ou plusieurs substituants identiques ou différents, qui sont choisis parmi OR⁸, NR^{9a}R^{9b}, halogène, aryle en C₆ à C₁₀ et hétaryle en C₃ à C₉ ;
R⁸ représentant l'hydrogène, un alkyle en C₁ à C₆, un aryle en C₆ à C₁₀, un aralkyle en C₇ à C₁₂ ou un alkylaryle en C₇ à C₁₂ ;
R^{9a}, R^{9b} signifiant chacun indépendamment l'un de l'autre l'hydrogène, un alkyle en C₁ à C₆, un aryle en C₆ à C₁₀, un aralkyle en C₇ à C₁₂ ou un alkylaryle en C₇ à C₁₂ ; ou R^{9a}, R^{9b} pouvant également signifier ensemble une chaîne alkylène de 2 à 5 atomes de carbone, qui peut être interrompue par N ou O ; et
R^{9c} représentant l'hydrogène, un alkyle en C₁ à C₆, un aryle en C₆ à C₁₀, un aralkyle en C₇ à C₁₂ ou un alkylaryle en C₇ à C₁₂.

8. Procédé selon l'une quelconque des revendications précédentes, dans lequel le composé de carbonyle α,β-insaturé prochiral est choisi parmi les composés de formules générales (IIa) et (IIb) 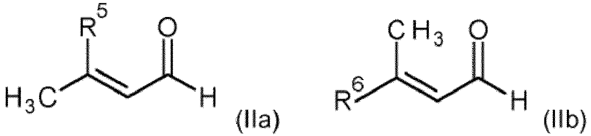 dans lesquelles R⁵, R⁶ représentent chacun un radical hydrocarboné non ramifié ou ramifié de 2 à 25 atomes de carbone, qui est saturé ou comprend 1, 2, 3, 4 ou 5 doubles liaisons éthyléniques non conjuguées.

9. Procédé selon la revendication 8 pour la fabrication de citronellal optiquement actif de formule (IV) 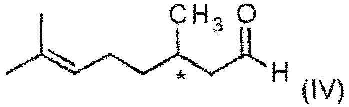 dans laquelle * désigne le centre d'asymétrie ;
par hydrogénation asymétrique de géranial de formule (IIa-1) ou de néral de formule (IIb-1)

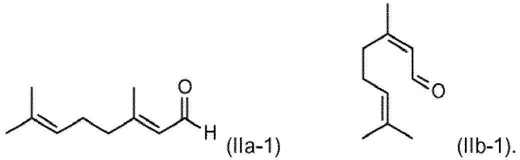

10. Procédé selon la revendication 9 pour la fabrication de D-citronellal par hydrogénation asymétrique de néral.

11. Procédé selon l'une quelconque des revendications précédentes, dans lequel le ligand bisphosphine bidentate chiral est un composé des formules générales (IX), (X) ou (XI) : 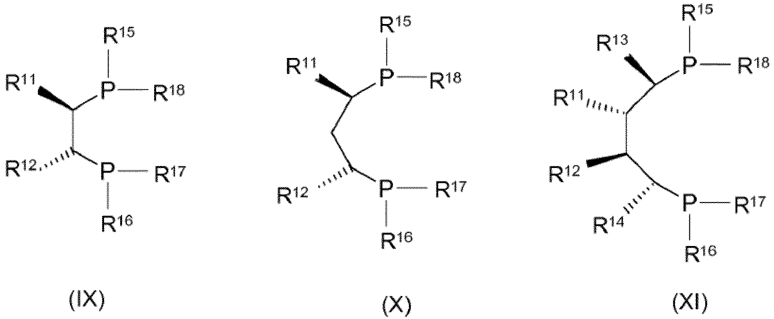 dans lesquelles
R¹¹, R¹² : représentent chacun indépendamment l'un de l'autre un radical hydrocarboné non ramifié, ramifié ou cyclique de 1 à 20 atomes de carbone, qui est saturé ou peut comprendre une ou plusieurs, généralement 1 à environ 4, doubles liaisons éthyléniques non conjuguées, et qui est non substitué ou porte un ou plusieurs, généralement 1 à 4, substituants identiques ou différents, qui sont choisis parmi OR¹⁹, NR²⁰R²¹, halogène, aryle en C₆-C₁₀ et hétaryle en C₃-C₉, ou
R¹¹ et R¹² peuvent également signifier ensemble un groupe alkylène de 2 à 10 chaînons ou un groupe cycloalkylène de 3 à 10 chaînons, dans lequel 1, 2, 3 ou 4 groupes CH₂ non voisins peuvent être remplacés par O ou N-R^{9c}, le groupe alkylène et le groupe cycloalkylène étant saturés ou comprenant une ou deux doubles liaisons éthyléniques non conjuguées, et le groupe alkylène et le groupe cycloalkylène étant non substitués ou portant un ou plusieurs substituants identiques ou différents, qui sont choisis parmi alkyle en C₁ à C₄ ;
R¹³, R¹⁴ : signifient chacun indépendamment l'un de l'autre l'hydrogène ou un alkyle en C₁ à C₄ linéaire ou ramifié, et
R¹⁵, R¹⁶, R¹⁷, R¹⁸ : sont identiques ou différents, et représentent un aryle en C₆ à C₁₀, qui est non substitué ou porte un ou plusieurs substituants, qui sont choisis parmi alkyle en C₁ à C₆, cycloalkyle en C₃ à C₆, aryle en C₆ à C₁₀, alcoxy en C₁ à C₆ et amino ;
R¹⁹, R²⁰, R²¹ : signifient chacun indépendamment les uns des autres l'hydrogène, un alkyle en C₁-C₄, un aryle en C₆-C₁₀, un aralkyle en C₇-C₁₂ ou un alkylaryle en C₇-C₁₂, R²⁰, R²¹ : pouvant également signifier ensemble une chaîne alkylène de 2 à 5 atomes de carbone, qui peut être interrompue par N ou O.

12. Procédé selon la revendication 11, dans lequel le ligand bisphosphine bidentate chiral est un composé de formule générale (IX).

13. Procédé selon la revendication 12, dans lequel le ligand bisphosphine bidentate chiral est le composé de formule (IXa) ou de formule (IXb) : 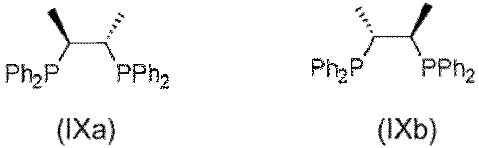 dans lesquelles Ph représente phényle.

14. Procédé selon la revendication 12, dans lequel le ligand bisphosphine bidentate chiral est le composé de formule (IXc) ou de formule (IXd) : 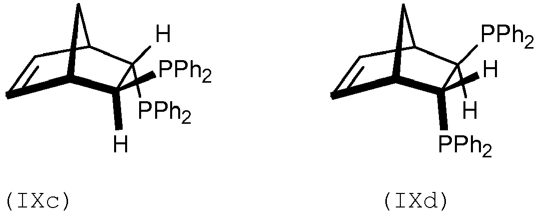 dans lesquelles Ph représente phényle.

15. Procédé selon l'une quelconque des revendications précédentes, qui présente au moins une ou toutes les caractéristiques a à g suivantes :
a) la réalisation de l'hydrogénation asymétrique à une pression d'hydrogène de 5 à 200 bar, notamment à une pression d'hydrogène de 10 à 100 bar ;
b) la réalisation continue du procédé :
c) la génération in situ du catalyseur avant ou pendant l'hydrogénation par mise en réaction d'un composé de rhodium achiral avec un ligand bisphosphine bidentate chiral ;
d) le prétraitement du catalyseur avant l'hydrogénation avec un mélange gazeux contenant du monoxyde de carbone et de l'hydrogène ;
e) la réalisation de l'hydrogénation asymétrique en présence de monoxyde de carbone en outre introduit dans le mélange réactionnel ;
f) la réalisation de l'hydrogénation asymétrique avec de l'hydrogène qui présente une teneur en monoxyde de carbone dans la plage allant de 50 à 3 000 ppm, notamment dans la plage allant de 100 à 2 000 ppm, spécialement dans la plage allant de 200 à 1 000 ppm et tout spécialement dans la plage allant de 400 à 800 ppm ;
g) la réalisation de l'hydrogénation asymétrique dans un réacteur à circulation de gaz ;
h) la réalisation de l'hydrogénation asymétrique dans un réacteur à circulation de gaz, le composé de carbonyle α,β-insaturé à mettre en réaction et l'hydrogène étant introduits dans le réacteur à circulation de gaz au moyen d'une buse à deux composants.

16. Procédé de fabrication de menthol optiquement actif comprenant les étapes suivantes :
i) la fabrication de citronellal optiquement actif par hydrogénation asymétrique selon la revendication 9 de géranial de formule (IIa-1) ou de néral de formule (IIb-1),
ii) la cyclisation du citronellal optiquement actif ainsi fabriqué en isopulégol optiquement actif en présence d'un acide de Lewis et
iii) l'hydrogénation de l'isopulégol optiquement actif ainsi fabriqué en menthol optiquement actif.
